# EUROPÄISCHE PATENTANMELDUNG

(11) **EP 4 685 133 A1**
(43) Veröffentlichungstag der Anmeldung: **28.01.2026**
(21) Anmeldenummer: 24191102.3
(22) Anmeldetag: 26.07.2024
(51) Int. Cl.: C07C 209/68, C07C 211/36, B01J 8/00

(54) **VERFAHREN ZUR KONTINUIERLICHEN, KATALYTISCHEN HYDRIERUNG VON MDA**

(71) Anmelder: Evonik Operations GmbH, 45128 Essen (DE)
(72) Erfinder: KUHLMANN, Hanns, 45549 Sprockhövel (DE); RIX, Armin Matthias, 45770 Marl (DE); PAUL, Niklas, 45770 Marl (DE); WESSNER, Lea, 44139 Dortmund (DE); VARGAS GÓMEZ, Maria, 45721 Haltern am See (DE); WINKLER, Tobias, 48249 Dülmen (DE); BOECK, Florian, 58455 Witten (DE); SUDHOFF, Daniel, 59192 Bergkamen (DE); LETTMANN, Christian, 48653 Coesfeld (DE)
(74) Vertreter: Evonik Patent Association

(57) **Zusammenfassung**

Anlage zur Hydrierung von Methylendianilin (MDA; Edukt1) mit einem Wasserstoffgeber (Edukt2), insb. einem gasförmigen Wasserstoffgeber, vorzugsweises Wasserstoff (H2), umfassend eine Konditionierungseinheit für die Edukte, eine Reaktoreinheit und eine Trenneinheit, wobei
- die Reaktoreinheit mind. einen Festbettreaktor als Hauptreaktor mit einer immobilen Katalysatorpackung umfasst, wobei der mind. eine (erste) Hauptreaktor, einen ersten Strömungsweg für das Stoffgemisch über die immobile Katalysatorpackung und einen weiteren Strömungswegumfasst, und wobei in den weiteren Strömungsweg ein Wärmetauscher eingebunden ist, zur Beeinflussung des Temperaturniveaus in dem ersten Strömungsweg;
- die Trenneinheit umfassend mind.
- eine erste Trennstufe, umfassend mind. einen Apparat zur Abtrennung des Lösungsmittels, wobei der mind. eine Apparat über mind. eine (Kopf-)Leitung mit mind. einem Kondensator stromabwärts verbunden ist, und
- eine zweite Trennstufe, umfassend mind. einen Apparat zur Abtrennung vom mind. einem Edukt und/oder mind. einem Nebenprodukt vom Produkt PACM, wobei der mind. eine Apparat über mind. eine (Kopf-)Leitung () mit mind. einem Kondensator stromabwärts verbunden ist, wobiei der weitere Strömungsweg ein geschlossener Medienumlauf für eine Wärmeträgermedium ist, der mind. auf einer Teilstrecke außerhalb der Katalysatorpackung des mind. einen Hauptreaktors zum indirekten Wärmeübergang verläuft, wobei in den Medienumlauf ein Wärmetauscher eingebunden ist.

## Beschreibung

Die Erfindung betrifft eine Anlage und ein Verfahren zur kontinuierlichen, katalytischen Hydrierung von MDA, insb. zur Herstellung von Methylenbis(cyclohexylamin), wie insb. 4,4'-Diaminodicyclohexylmethan (PACM).

Verfahren zur Hydrierung organischer Verbindungen, insbesondere zur Hydrierung von aromatischen Verbindungen zu den entsprechenden Cyclohexan-Derivaten sind aus dem Stand der Technik bereits bekannt.

Methylenbis(cyclohexylamin) ist ein bei Standardbedingungen (SATP) festes oder flüssig vorliegendes cycloaliphatisches Amin, das üblicherweise über die Flüssigphasen-Hydrierung von MDA hergestellt wird. Das Akronym MDA wurde historisch als Abkürzung für das bei der Umsetzung von Anilin und Formaldehyd gebildete, vor allem "Methylendianilin" (Diaminodiphenylmethan) umfassende Produktgemisch eingeführt und wird weiterhin zur Bezeichnung des mittlerweile großtechnisch erzeugten Verfahrensproduktes verwendet. Das Produkt der Hydrierung, das vor allem Methylenbis(cyclohexylamin) umfasst, wird deswegen oft auch als H12MDA bezeichnet.

MDA ist aufgrund seines Herstellungsprozesses üblicherweise ein Gemisch aus verschiedenen Diaminodiphenylmethanen. Vor allem besteht es aus 4,4`-Diaminodiphenylmethan. Es können jedoch auch 2,4`- und 2,2'-lsomere vorliegen. MDA kann weiterhin bei der Umsetzung von Anilin und Formaldehyd gebildete Reaktionsprodukte mit drei oder mehr aromatischen Ringen, insbesondere solche mit drei oder mehr Phenylringen, enthalten. Diese Reaktionsprodukte mit drei oder mehr aromatischen Ringen werden auch als Mehrkernverbindungen bezeichnet.

Bei dem im Handel erhältlichen Methylenbis(cyclohexylamin) handelt es sich aufgrund des hohen Anteils an 4,4`-Diaminodiphenylmethan im eingesetzten MDA größtenteils um 4,4'-Diaminodicyclohexylmethan bzw. Bis(para-Aminocyclohexyl)methan. Aufgrund der potentiellen Anwesenheit der entsprechenden 2,4'- und 2,2'-Diaminophenylmethan-lsomere im MDA kann in Methylenbis(cyclohexylamin) auch 2,4`-Diaminodicyclohexylmethan und 2,2'-Diaminodicyclohexylmethan vorliegen. Weiterhin kann hydriertes MDA neben Methylenbis(cyclohexylamin) weiterhin noch (ggf. partiell) hydrierte Mehrkernverbindungen enthalten.

US 5,578,546 A offenbart, dass 1947 erstmalig ein Verfahren zur Herstellung von Methylenbis(cyclohexylamin) beschrieben und 1965 in den technischen Maßstab überführt wurde. Die Hydrierung von MDA ist stark exotherm. So gibt WO 2010/069484 A1 eine Reaktionsenthalpie von -1600 kJ/mol an.

In Folge der Hydrierung werden je nach Verfahren verschiedene Diastereoisomere gebildet. Dabei kann das von 4,4`-Diaminodiphenylmethan abgeleitete Produkt 4,4'-Diaminodicyclohexylmethan (PACM) als trans/trans-, cis/cis- und cis/trans-Isomer vorliegen und ist deswegen in der Regel ein Gemisch dieser Isomere mit unterschiedlichen Anteilen. Mit steigendem trans/trans-Gehalt steigt dabei der Schmelzpunkt der Verbindung. Deshalb unterscheiden sich die Einsatzgebiete je nach Isomerengehalt stark: Während Methylenbis(cyclohexylamin)-Qualitäten mit niedrigem trans/trans - Gehalt (z.B. 10-30 Gew.-%) im Bereich der Amin- und Isocyanat-Vernetzer, insbesondere im Bereich von Zwei-Komponenten-Harzen Einsatz finden, finden Qualitäten mit hohem trans/trans - Gehalt (z.B. ≥ 48 Gew.-%) vor allem Anwendung als Regulator in Polyamidverbindungen. Gerade die Herstellung von Produkten mit niedrigem trans/trans-Gehalt stellt eine Herausforderung dar, da das thermodynamische Gleichgewicht, wie in US 3,636,108 A beschrieben wird, im Bereich wesentlich höherer trans/trans-Anteile (bis zu 51,2 %) liegt. US 2,606,925 A zeigt zudem, dass das Gleichgewicht nachträglich durch längeres Temperieren in Richtung eines höheren Anteils an trans/trans-Isomer verschoben werden kann.

Die Zusammensetzung des Hydrierungsproduktes hängt auch von der Zusammensetzung des eingesetzten MDAs ab: Oft wird MDA in Qualitäten von MDA50 bis MDA100 eingesetzt, wobei die zwischen 50 und 100 liegende Zahl den Gehalt an Diaminodiphenylmethanen im MDA-Gemisch angibt. MDA50 ist dabei eine MDA-Qualität, die wie oben erläutert prozessbedingt etwa 50 Gew.-% Diaminodiphenylmethanen und 50 Gew.-% Mehrkernverbindungen enthält. Die einzelnen Mehrkernverbindungen können entsprechend der enthaltenen Aromatenanzahl als 3-Kern-Verbindungen, 4-Kern-Verbindungen, etc. bezeichnet werden. MDA50 ist dabei die am meisten produzierte Qualität und wird hauptsächlich zu Methylendicyclohexyldiisocyanat (MDI) weiterverarbeitet. MDA100 ist reines MDA bzw. Diaminodiphenylmethan ohne Mehrkernverbindungen. MDA85 oder MDA90 sind andere auf dem Markt verfügbare Qualitäten mittlerer Reinheit. Wenn in Patentschriften zum Herstellverfahren von Methylenbis(cyclohexylamin) auf die Reinheit der MDA-Qualität eingegangen wird, so ist meist von MOA 100 die Rede (z.B. CN 110204447 B). Dagegen stellt US 2005/261525 A1 bevorzugt auf die Hydrierung von MDA50 ab. Die dabei als Hochsieder anfallenden hydrierten, oligomeren Amine eignen sich als Vernetzer mit besonders niedrigen Dampfdrücken für eine Reihe von Spezialanwendungen, wie US 2004/162409 A1 zeigt.

Der Gehalt an (ggf. partiell) hydrierten Mehrkernverbindungen im Produkt nimmt in der Reihenfolge der Edukte MDA50, MDA85, MDA90, MDA100 ab, da der Gehalt an Mehrkernverbindungen von MDA50 zu MDA100 abnimmt.

Aus der WO 2009/153123 A1 ist ein kontinuierliches Verfahren und einen Reaktor zur Hydrierung organischer Verbindungen in einem mehrphasigen, mehrstufigen System in Gegenwart eines homogenen oder heterogenen Katalysators bekannt. Als Katalysatoren werden u.a. Edelmetalle, wie Platin, Palladium, Ruthenium und Rhodium oder andere Übergangsmetalle, wie Molybdän, Wolfram und Chrom vorgeschlagen. Hierbei können die heterogenen Katalysatoren auf Trägermaterialien angeordnet sein, wie bspw. Kohlenstoff, Aluminiumoxid, Siliciumdioxid, Zirkoniumdioxid, Zeolithe, Alumosilikate oder Mischungen dieser Trägermaterialien. Als Substrate werden in diesem Verfahren bevorzugt aromatische Verbindungen enthaltend Amino-Substituenten eingesetzt, beispielsweise MDA, Polymer-MDA, Anilin, 2,4-Diaminotoluol, 2,6-Diaminotoluol, o-Phenylendiamin, etc. Die heterogenen Katalysatoren werden in Suspension eingesetzt.

DE 19533718 A1 offenbart ein Verfahren zur Hydrierung von aromatischen Verbindungen, in denen mindestens eine Aminogruppe an einen aromatischen Kern gebunden ist. Dazu kann ein heterogener Katalysator enthaltend Ruthenium und gegebenenfalls mindestens ein Metall der I-, VII- oder VIII-Nebengruppe verwendet werden. Als Trägermaterial wird beispielsweise Aluminiumoxid, Siliciumdioxid, Titandioxid oder Zirkoniumdioxid, vorzugsweise Aluminiumdioxid oder Zirkoniumdioxid, eingesetzt. Als Beispiel angegeben wird lediglich ein Katalysator enthaltend Ruthenium auf dem Trägermaterial Aluminiumoxid, nicht jedoch Zirkoniumoxid.

EP 1337331 A1 offenbart ein Verfahren zur katalytischen Hydrierung von aromatischen oder heteroaromatischen Aminen, wobei als Aktivmetall Ruthenium fungiert und der Katalysator mindestens ein weiteres Metall der I-, VII-, oder VIII-Nebengruppe enthält und diese auf einem Trägermaterial aufgebracht sind. Hierbei werden als aromatische Verbindungen u.a. 4,4'-MDA und Isomeren hiervon eingesetzt. Auch die EP 0111238 A1 offenbart ein Verfahren zur katalytischen Hydrierung von 4,4'-MDA, dadurch gekennzeichnet, dass die Hydrierung in Gegenwart von geträgertem Ruthenium in Anwesenheit von Nitraten und Sulfaten der Alkalimetalle und Nitraten der Erdalkalimetalle erfolgt. Ein vergleichbares Verfahren wird in der EP 1366812 A1 offenbart, wobei als Trägermaterialien unter anderem Aluminiumoxid, Siliciumoxid, Titanoxid und Zirkoniumoxid genannt werden.

Weitere Verfahren zur Hydrierung organischer Verbindungen offenbaren WO 2011/003899 A1 und WO 2009/090179 A1. Hier werden Verfahren zur Hydrierung von aromatischen Aminen mit Wasserstoff in Gegenwart eines Ru-Katalysators, welcher, u.a. Zirkoniumoxid-Trägermaterial enthält, offenbart.

Schließlich ist aus der EP 2 883 863 B1 ein Verfahren und eine Anlage zur Hydrierung von 4,4'-Methylendianilin (MDA) und/oder Polymer-MDA mit Wasserstoff in Gegenwarteines Katalysators bekannt. Hierbei wird als Katalysator Ruthenium vorgeschlagen, welcher auf einem Zirkoniumoxid-Trägermaterial aufgebracht ist. Bezüglich des Reaktors, verweist die EP 2 883 863 B1 auf den Reaktor bzw. das Reaktorkonzept der WO 2008/015135 A1. Aus dieser WO 2008/015135 A1 ist ein kontinuierliches Verfahren und eine Anlage zur Hydrierung von Diisononylphthalat zu 1,2-Cyclohexandicabonsäurediisononylester (DINCH) bekannt, wobei DINP als Gemisch in einem organischen Lösungsmittel, mit Wasserstoff bei einem Druck von bis zu 325 bar hydriert wird. Hierbei wird eine in Reihenschaltung von zwei Festbettreaktoren mit jeweils einer immobilen Festbettschüttung vorgeschlagen. Zur Ableitung der Reaktionswärme aus den beiden Reaktoren wird vorgeschlagen, einen Teilstrom des Stoffgemisches nach dem zweiten Festbettreaktor zu rezirkulieren und diesen hierbei zu kühlen. Ein vergleichbares Reaktorkonzept von in Reihe geschalteten Festbettreaktoren zur Hydrierung von ist auch aus der EP 1 566 372 B1 bekannt.

Nachteilig an dem Konzept ist, dass die Kreislaufführung eines Produktteilstroms zu einer erhöhten Bildung von unerwünschten Nebenprodukten führen kann und die Anlagenleistung senkt, wobei erhöhte Energiekosten für die Rückführung und Kühlung des Recyclingstroms erzeugt werden und insgesamt der Betrieb der Anlage und das Verfahren sehr energieintensiv sind.

Wie ausgeführt, ist der Bedarf an bspw. PACM mit unterschiedlichen Anteilen der jeweiligen Isomeren abhängig vom dem Einsatzzweck bzw. den nachfolgenden Produkten. So sind beispielsweise PACM-Qualitäten mit niedrigem trans/trans-Gehalt von 10 bis 30 Gew.% im Bereich der Amin- und Isocyanat-Vernetzer bevorzugt, insbesondere im Bereich der Formulierung von 2-Komponenten-Harzen, wobei PACM-Qualitäten mit hohem trans/trans-Gehalt von über 48 Gew.% vor allem Anwendung als Regulator in Polyamidverbindungen finden. Die genannten Gew.% beziehen sich hierbei auf das PACM-Isomerengemisch als solches. Gerade die Herstellung von Produkten mit niedrigem trans/trans-Gehalt stellt eine verfahrenstechnische Herausforderung dar, da das thermodynamische Gleichgewicht, wie in US 3,636,108 A beschrieben wird, im Bereich wesentlich höherer trans/trans-Anteile von bis zu 51,2 Gew.% liegt. Weiterhin ist aus der US 2,606,925 A bekannt, dass das Gleichgewicht der PACM-Isomere durch längeres Temperieren nachträglich in Richtung zu einem höheren Anteil an trans/trans-Isomer verschoben wird.

Aufgabe der vorliegenden Erfindung ist es somit, eine verbesserte Anlage und einen verbesserten Prozess bereitzustellen, der hinsichtlich Produktumsatz und Energieeffizienz verbessert ist und insb. die Herstellung definierter Anteile der jeweiligen Isomere im Isomerengemisch ermöglicht.

Die Aufgabe wird erfindungsgemäß mit einer Anlage nach den Merkmalen des Anspruch 1 und einem Verfahren nach den Merkmalen des Anspruchs 16 gelöst.

Hierbei wird eine Anlage vorgesehen, zur kontinuierlichen, katalytischen Hydrierung von Methylendianilin (MDA; Edukt1) mit einem Wasserstoffgeber (Edukt2), insb. einem gasförmigen Wasserstoffgeber, vorzugsweises Wasserstoff (H2), umfassend eine Konditionierungseinheit (104) für die Edukte, eine Reaktoreinheit (102) und eine Trenneinheit (106), wobei
- die Konditionierungseinheit (Zu-)Leitungen für Edukte1, Edukt2 und mind. ein Lösungsmittel, mind. einen Wärmetauscher in mind. einer (Zu-)Leitung, mind. einen Mischer zur Mischung der Edukte und/oder mind. eines Edukts mit mind. einem Lösungsmittel umfasst;
- die Reaktoreinheit mind. einen Festbettreaktor als Hauptreaktor mit einer immobilen Katalysatorpackung umfasst, wobei der mind. eine Hauptreaktor einen ersten Strömungsweg für das Stoffgemisch über die immobile Katalysatorpackung, wobei der mind. einen Hauptreaktor in einen weiteren Strömungsweg eingebunden ist, und wobei in den weiteren Strömungsweg ein Wärmetauscher eingebunden ist, zur Beeinflussung des Temperaturniveaus in dem ersten Strömungsweg;
- die Trenneinheit umfassend mind.
- eine erste Trennstufe, umfassend mind. einen Apparat zur Abtrennung des Lösungsmittels, wobei der mind. eine Apparat über mind. eine (Kopf-)Leitung mit mind. einem Kondensator stromabwärts verbunden ist, und
- eine zweite Trennstufe, umfassend mind. einen Apparat zur Abtrennung vom mind. einem Edukt und/oder mind. einem Nebenprodukt vom Produkt, wobei der mind. eine Apparat über mind. eine (Kopf-)Leitung mit mind. einem Kondensator stromabwärts verbunden ist, **wobei**
der weitere Strömungsweg ein geschlossener Medienumlauf für ein Wärmeträgermedium ist, der mind. auf einer Teilstrecke außerhalb der Katalysatorpackung des mind. einen Hauptreaktors zum indirekten Wärmeübergang verläuft, wobei in den Medienumlauf ein Wärmetauscher eingebunden ist.

Der weitere Strömungsweg ist insb. derart mit den sonstigen Leitungen der Anlage und insb. der Reaktoreinheit verknüpft, dass im Regelbetrieb kein Edukt- oder Stoffgemisch hierin strömen kann.

Durch diesen weiteren Strömungsweg für einen geschlossenen Medienumlauf, in den vorteilhafterweise eine Pumpe als Fördermittel eingebunden ist, kann im Vergleich zur WO 2008/015135 A1 eine wesentliche Leistungssteigerung erreicht werden. Weiterhin überraschenderweise durch eine quasi isotherme Betriebsweise des Hauptreaktors eine trans/trans-Verhältnis im Produkt von 22 Gew.% erreicht werden.

Die Anlage dient vorzugsweise zur kontinuierlichen, katalytischen Herstellung von Methylenbis(cyclohexylamin) als Produkt, insb. zur Produktion von 4,4'-Diaminodicyclohexylmethan (PACM), nach der Formel (I)

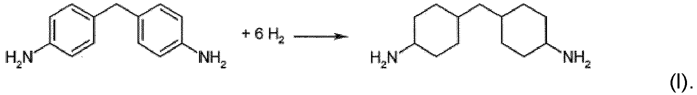

Gemäß einer weiteren Ausführungsform der Anlage kann es vorteilhaft sein, wenn die Reaktoreinheit einen ersten Hauptreaktor und mind. einen stromabwärts befindlichen seriell verbundenen weiteren Nachreaktor umfasst.

Der Nachreaktor ist vorteilhafterweise seriell zum Hauptreaktor geschaltet und weist einen immobilen Katalysator auf, der stofflich identisch oder weitgehend identisch zu dem des Hauptreaktors sein kann. Die Menge und räumliche Anordnung des immobilen Katalysators, sowie der Strömungsweg des Stoffstroms innerhalb des Nachreaktors sind hierbei derart gewählt, dass maximal 20% des molaren Umsatzes an MDA, vorteilhafterweise maximal 15%, idealerweise maximal 10%, wobei bei 100% an molarem MDA-Umsatz, die Reaktion als abgeschlossen gilt. Der Nachreaktor wird vorteilhafterweise als adiabater Reaktor betrieben, wobei gemäß einer weiteren Ausführungsform der Anlage es vorteilhaft sein kann, wenn in der Leitung zwischen dem mind. einen Hauptreaktor und dem Nachreaktor mind. ein Wärmetauscher angeordnet ist.

Mit fortschreitender Zeit erfolgt ein Absinken der Katalysatoraktivität bis ein Austausch oder eine Regenerierung erforderlich ist. Hierzu wird parallel durch Regelung des Kühlkreislaufs die Betriebstemperatur angehoben, d.h. insb. eine geringere Kühlung über den im Kühlkreislauf eingebundenen Wärmetauscher vorgenommen, um Umsatz und Selektivität insgesamt auf einem weitgehend gleichbleibenden Niveau zu halten. Dies hat zur Folge, dass das Isomerenverhältnis verschoben wird, hinzu höheren trans/trans-Anteilen im Produkt. Hierbei hat es sich als sehr vorteilhaft erwiesen, die Temperatur im Feed des Nachreaktors über den vorlaufenden Wärmetauscher eigenständig geregelt werden kann, insb. gleichläufig. So wird mit steigender Betriebstemperatur des Hauptreaktors über die Nutzungszeit des Katalysators die Feedtemperatur im Stoffstrom des Nachreaktors abgesenkt.

Mit dem separaten Nachreaktor, insb. dem adiabatischen Nachreaktor, ist eine optimierte Steuerung des Prozesses und der Anlage möglich geworden, wodurch die Selektivität durch vom Hauptreaktor (Auslass) abweichende Eintrittstemperatur des Nachreaktors möglich wird. Typischerweise kann die Eintrittstemperatur des Nachreaktors bei der gleichen oder einer geringfügig niedrigeren Temperatur vorgesehen werden, die bis zu 30 °C unterhalb der Auslasstemperatur des Hauptreaktors liegen kann. Somit kann mind. zeitweise eine Temperaturerhöhung im (adiabatischen) Nachreaktor von 20 °C bis 40 °C, typischerweise von 20 °C bis 30 °C zugelassen werden, und so gezielt die gewünschte Produktqualität (Isomerenverhältnis) eingestellt werden. Auf diese Weise ist ein sehr niedriger und ein sehr genauer Anteil an trans/trans Isomeren in dem Isomerengemisch einstellbar. Der Hauptreaktor kann auf einem möglichst niedrigen Temperaturniveau betrieben werden, so dass der trans/trans-Anteil des PACM im Stoffstrom (Auslass des Hauptreaktors) ca. 13 bis 20 Gew.% beträgt, wobei ca. 13 % bei einem neuen bzw. regenerierten Katalysator erreichbar sind und 20 Gew.% bei einem Katalysator nach Langzeitnutzung (kurz vor Austausch/Regeneration). Abhängig von der Phase, in der sich der Hauptreaktor befindet, kann es mind. zeitweise sinnvoll sein, wenn die Eintrittstemperatur des Nachreaktors um 5 bis 20 °C höher als die des Hauptreaktors ist.

Auch wenn der Hauptreaktor vorliegend als "isotherm/-isch" bezeichnet wird, wird dieser ideale Zustand bei industrieller Anwendung nur bedingt erreicht, so dass sich innerhalb des Hauptreaktors wegen unvollständigem Wärmeabtransport ein Temperaturgradient von ca. 5 bis 10 °C in radiale und auch in Strömungsrichtung ausbildet.

Der Nachreaktor wird über den vorlaufenden Wärmetauscher mit einer etwas höheren Temperatur derart beschickt, dass die gewünschte restliche Reaktion und Isomerenumformung zu dem finalen, gewünschten trans/trans-Verhältnis von bspw. 17 bis 23 Gew.% erfolgt. Der Nachreaktor mit der dortigen schwach exothermen, restlichen Reaktion wird hierbei weitgehend adiabat betrieben, was aber nicht im idealen Sinne zu verstehen ist.

Vom Zeitpunkt t₀, dem Verfahrensstart nach Erneuerung oder Regeneration des Katalysators bis zum Zeitpunkt t₄, dem Ende des Verfahrens, bestimmt durch die Erneuerung oder Regeneration des Katalysators, kann die Betriebstemperatur des Hauptreaktors erhöht und Temperatur im Stoffstrom im Einlass (Feed) des Nachreaktors gleichgehalten oder gesenkt werden. Vorteilhafterweise ist die Anlage so ausgebildet, dass die Temperaturerhöhung oder -Absenkung kontinuierlich und/oder schrittweise erfolgen kann.

Der Hauptreaktor wird hierbei isotherm oder weitgehend isotherm und der Nachreaktor adiabat oder weitgehend adiabat betrieben. Zum Zeitpunkt t₀ dem Verfahrensstart kann neben dem Hauptreaktor auch der Katalysator im Nachreaktor erneuert oder regeneriert worden sein. Vorteilhafterweise wird der Zyklus zur Regeneration des Nachreaktors eigenständig und vom Hauptreaktor unabhängig bestimmt, insb. wenn o.g. Zusammenwirken der beiden Reaktoren nicht mehr die Produktion des gewünschten niedrigen trans/trans-Anteils im PACM ermöglicht.

Der von einem Druckentspannungstrennkessel, nachstehend Trennkessel genannt (auch teilweise Flashbehälter genannt), kommende flüssige Stoffstrom wird über eine Leitung zur ersten Trennkolonne innerhalb der ersten Trennstufe der Trenneinheit geleitet. Der Trennkessel zeichnet sich dadurch aus, dass durch Entspannung (Druckabsenkung) der zufließende Stoffstrom in eine Dampfphase (Lösungsmittel, lösungsmittelreich) und eine Flüssigphase (verarmt an Lösungsmittel) aufgetrennt wird und im Regelbetrieb im Trennkessel beide Phasen vorliegen. Der Trennkessel kann zusätzlich einen Sumpfumlauf mit integriertem Wärmetauscher aufweisen oder hieran angeschlossen sein, um durch Erwärmung der Flüssigphase den abtrennbaren Dampfanteil über den druckbedingten Anteil hinaus zu erhöhen. Weiterhin kann ein Trennkessel Einbauten oder Füllkörper umfassen, insb. um das Mitreißen von nicht oder nur unvollständig an Lösungsmittel verarmten Tröpfchen zu verhindern. Vorteilhafterweise kann bei einer Ausführungsform der Anlage vorgesehen sein, dass in der Leitung vom Trennkessel zur ersten Kolonne eine Druckregeleinheit vorgesehen ist. Hierdurch kann die Reaktoreinheit auf einem ersten, hohen Druckniveau betrieben werden und die ersten Trennstufe der Trenneinheit auf einem zweiten, tieferen Druckniveau. Im vorliegenden Zusammenhang meint also Trennkessel (Flashbehälter) einen Apparat, indem im Wesentlichen durch Druckentspannung eine Phasentrennung veranlasst wird. Trennkolonne meint vorliegend hingegen einen Apparat, in dem im Wesentlichen durch Energiezufuhr einen Trennung in eine Dampfphase und Flüssigphase erfolgt, insb. unter Einbindung eines Kopfumlaufs, indem im Kopf mind. ein Teil der auskondensierten Flüssigkeit zurück in die Kolonne geleitet wird. Vorteilhafterweise ist die Mischereinheit zweiteilig ausgebildet und umfasst bspw. einen Mischapparat und einen Gas-Sättiger. Der Mischapparat kann insb. ein dynamischer oder statischer Mischer sein, der zur innigen Verbindung von MDA (Edukt1) mit dem Lösungsmittel geeignet ist. Der Gas-Sättiger kann insb. eine kleine Kolonne oder ein Kessel sein, in dem geeignete Einbauten vorhanden sind, um das unter einem hohen Druck zugeführte H2-Gas, intensiv im MDA-Lösungsmittel-Stoffstrom zu lösen und/oder homogen vor dem Eintritt in den Hauptreaktor zu verteilen. Der H2-Gasdruck beträgt vorteilhafterweise 70 bar bis 100 bar.

Mit dem Begriff "immobile Katalysatorpackung" oder "immobiler Katalysator" ist jede Form eines lokalen, nicht mit dem Stoffstrom strömenden oder fließenden Katalysator gemeint, wie insb. Katalysatorschüttungen (Pellets oder beschichtete Trägerkörper) oder feste Einbauten, die mit Katalysatormaterial beschichtet sind. Vorteilhafte Einbauteile, die eine Katalysatorbeschichtung aufweisen, können bspw. Gitter, Platten oder sonstigen Köper sein, die im Hauptreaktor angeordnet sind.

Im vorliegenden Zusammenhang meint eine "XY-einheit" und/oder "XY-stufe" immer auch mind. einen entsprechenden Apparat, Vorrichtung oder dergleichen, der/die von der jeweiligen Einheit oder Stufe umfasst ist. So meint bspw. eine Mischeinheit/-stufe, dass diese mind. einen Mischer/- vorrichtung umfasst.

Im vorliegenden Zusammenhang meint "Wärmetausch" oder "Wärmetauscher" immer einen indirekten Wärmetausch und entsprechende Bauformen mit geschlossenen Stoff- und Medienführungen, es sei denn, es ist ausdrücklich etwas hiervon Abweichendes beschrieben.

Mit der "Kondensationseinheit" der ersten Trennstufe ist ein einzelner Wärmetauscher oder eine Gruppe von Wärmetauschern bezeichnet, die zur mind. teilweisen Kondensation und/oder Abkühlung der über die Kopfleitung/en abgeleiteten Anteile an Leichtsiedern dienen. Eine derart bezeichnete "Kondensationseinheit" muss keine (geschlossene) Baueinheit darstellen. Somit wird zum Teil der Begriff "Kondensationseinheit" und einen einzelner "Wärmetauscher" auch synonym verwendet. Ein "Kondensator" meint einen Wärmetauscher, der kühlend auf eine (Dampf-)Leitung einwirkt und eine mind. teilweise Kondensation des Dampfes in der entsprechenden Leitung veranlassen soll. Eine solche Benennung ist allerdings nicht einschränkend zu verstehen, weil im vorliegend Zusammenhang zur EK alle Medienströme, Edukt- und Stoffströme betrachtet werden, so dass bspw. eine Kondensator als Wärmequelle dienen kann, für einen gekoppelten, stromabwärtsbefindlichen Wärmetauscher. Das finale Verständnis des Gemeinten muss somit immer aus dem jeweiligen Textzusammenhang genommen werden.

Die als "erste Trennstufe" bezeichnete Stufe ist insb. dadurch bestimmt und weist entsprechende Apparate und Leitungen auf, dass das Lösungsmittel (gezielt) vom produktreichen Stoffstrom abzutrennen und vorteilhafterweise auch zur Verwendung in die Reaktoreinheit und/oder die Konditionierungseinheit zurückgleitet wird. Die erste Trennstufe ist dadurch bestimmt, dass mind. 80%, idealerweise mind. 90 % des Lösungsmittels abgetrennt wird. In analoger Weise meint die als "zweite Trennstufe" bezeichnete Stufe der Trenneinheit, dass diese dadurch bestimmt ist und entsprechende Apparate und Leitungsführungen aufweist, um das Produkt von Nebenprodukten und Edukten, insb. MDA zu trennen und das Produkt aufzureinigen. Hierbei sind die erste und zweite Trennstufe ggf. nicht ganz streng getrennt und es kann ein Überlappungsbereich oder ein Apparat im Übergangsbereich vorhanden sein, in dem sowohl Lösungsmittel als auch mind. ein Nebenprodukt oder mind. ein Edukt vom Produkt getrennt wird. In dem vorliegenden Zusammenhang meint die "Abtrennung von Lösungsmittel" in der ersten Trennstufe und "Abtrennung von mind. einem Edukt und/oder mind. einem Nebenprodukt vom Produkt", wobei das Produkt insb. PACM sein kann, dass die Abtrennung keine absolute Abgrenzung der Trennstufen meint, sondern jeweils "im Wesentlichen" nur den/die genannten Stoff/e betrifft.

Der Begriff "Eduktmischung" meint in dem vorliegenden Zusammenhang die Mischung aus Stoffen, die beim Eintritt in den (ersten) Hauptreaktor vorliegt, also die Mischung aus allen Edukten, Lösungsmitteln, Hilfsstoffen etc. In und nach dem mind. einen Reaktor wird die strömende Mischung aus Stoffen in jedem Grad der Reaktion oder nachfolgenden Aufreinigung als "Stoffstrom" oder "Stoffmischung" (auch "Stoffgemisch") bezeichnet, wobei zum Teil adjektivische Beschreibungen wie "produktreich" oder "lösungsmittelreich" angefügt sind. Die stoffliche Zusammensetzung des Stoffstroms an jedem Ort der Anlage ergibt sich für den Fachmann allerdings auch in naheliegender Weise durch ebendiesen Anlagenort und stromaufwärts befindliche Anlagenkomponenten und insb. die verfahrenstechnischen Apparate der Anlagen. Die Reinstoffe, wie bspw. das Produkt PACM, sowie die Nebenprodukte LB und HB, sind gesondert benannt und ausgewiesen. Hierbei steht "LB" für "Low Boiler", einem werthaltigen Stoffgemisch, das gesondert aus dem Stoffstrom und vom Produkt abgetrennt wird und einen niedrigen Siedepunkt von ca. 240 °C bis 290 °C aufweist. In analoger Weise steht "HB" für "High Boiler", einem werthaltigen Stoffgemisch, das gesondert aus dem Stoffstrom und vom Produkt abgetrennt wird und einen hohen Siedepunkt von > 350 °C aufweist.

Vorliegend wird insb. eine Anlage und ein Verfahren zur Produktion von Methylenbis(cyclohexylamin) als Produkt, insb. zur Produktion von 4,4'-Diaminodicyclohexylmethan (PACM), durch katalytische Hydrierung von Methylendianilin beansprucht. Hierbei ist das primäre Produkt PACM, das aus 4,4'-Diaminodiphenylmethan (4,4'-MDA; primärer Anteil des Edukts1) hydriert wird, insb. mit dem genannten niedrigen trans/trans-Isomerenverhältnis, so dass die Anlage und das Verfahren insb. zur Produktion von 4,4'-Diaminodicyclohexylmethan (PACM) durch kontinuierliche, katalytische Hydrierung von 4,4'-Diaminodiphenylmethan (4,4'-MDA) dient und geeignet ist. Die weiterhin vorliegenden Anteile 2,4'-MDA und 2,2'-MDA des MDA werden mind. zu geringen Anteilen parallel zu Reaktionsprodukten gewandelt, wobei diese in der Regel in der Produktmischung verbleiben. Weiterhin können vorteilhafterweise ebenfalls durch dieses Verfahren bzw. diese Anlage in der zweite Trennstufe der Trenneinheit sonstige Nebenprodukte aufgereinigt und abgetrennt werden, insb. in mind. einer Trennkolonne. Diese stellen sekundäre (werthaltige) Produkte dar und sind hierin im Wesentlichen als High Boiler (HB) und Low Boiler (LB) beschrieben und gemeint. Gemäß einer vorteilhaften Ausführungsform des Verfahrens, beträgt der

Vorteilhafterweise ist das Lösungsmittel aus der nachfolgenden Gruppe von Stoffen: Cyclohexan, Dioxan, Tetrahydrofuran (THF), Cyclohexylamin, dicyclohexylamin, Methanol, Ethanol, Isopropanol, n-Butanol, 2-Butanol, 2-Methoxy-2-methylpropan (MTBE) oder Methylcyclohexan oder ein Gemisch hieraus. Vorteilhafterweise wird das Lösungsmittel, insb. THF im Überschuss zum MDA zugeführt, so dass vorteilhafterweise das Verhältnis der Massenströme von Lösungsmittel, insb. THF, zu MDA am Einlass des Hauptreaktors im Bereich von 1,0 bis 8,0 liegt, insbesondere im Bereich von 2,0 bis 7,5.

In dem vorliegenden Zusammenhang werden unterschiedliche Formen der energetischen Kopplung und Wärmeübertragung beschrieben, die wie folgt definiert sind:
Insgesamt kann es vorteilhaft sein, wenn die erste Trennstufe der Trenneinheit mind. einen Trennkessel (Trennkessel) umfasst, der mit dem Hauptreaktor, dem Nachreaktor oder dem in Strömungsrichtung letzten Hauptreaktor über eine Leitung verbunden ist, wobei der Trennkessel einen Kopfauslass, einen Boden-/Sumpfauslass und einen beheizbaren Sumpfumlauf mit mind. einem Wärmetauscher umfasst, wobei in die (Kopf-)Leitung, die an den Kopfauslass angeschlossen ist, die mind. eine Kondensationseinheit eingebunden ist. Weiterhin kann stromabwärts der Kondensationseinheit ein Sammelbehälter und/oder eine Verbindungseinheit/- knoten mit/in die (Rück-)Leitung für das Lösungsmittel angeordnet sein.

Der von dem Trennkessel kommende Stoffstrom wird über eine (Feed-)Leitung zur ersten Trennkolonne der zweite Trennstufe der Trenneinheit geleitet. Vorteilhafterweise kann bei einer Ausführungsform der Anlage vorgesehen sein, dass in der (Feed-)Leitung vom Trennkessel zur ersten Kolonne eine Druckregeleinheit vorgesehen ist. Hierdurch kann die Reaktoreinheit auf einem ersten, hohen Druckniveau betrieben werden und die erste Trennstufe der Trenneinheit auf einem zweiten, tieferen Druckniveau.

Vorteilhafterweise kann vorgesehen werden, dass mind. eine EK vorgesehen ist, in die mind. einer der folgenden Wärmetauscher eingebunden ist:
- der mind. eine Kondensator der ersten Trennstufe, insb. des Kondensators in der (Kopf-)Leitung des eines Trennkessels,
- der mind. eine Kondensator der zweiten Trennstufe, insb. ein Kondensator einer Trennkolonne der zweiten Trennstufe,
- der mind. eine Wärmetauscher des Medienumlaufs des mind. einen Hauptreaktors.

Hierbei ist mit einer Energiekopplung (EK) eine integrierte Energiekopplung oder eine direkte Energiekopplung gemeint, wobei
i) integrierte EK eine integrierte Energiekopplung meint, unterteilt in
   a. eine integrierte stoffbasierte Energiekopplung (integrierte stoffbasierte EK) von mind. zwei Stoffströmen in einem Wärmetauscher im indirekten Wärmeaustausch meint, d.h. eine bauliche Integration in einem einzigen Wärmetauscher (Apparat), d.h. wo vormals zwei Wärmetauscher vorgesehen waren, sind beide Wärmetransportaufgaben einer baulichen Einheit (Wärmetauscher) integriert,
   b. eine integrierte medienbasierte Energiekopplung (integrierte medienbasierte EK) von mind. zwei Wärmetauschmedien in einem Wärmetauscher im indirekten Wärmeaustausch meint, d.h. eine bauliche Integration in einem einzigen Wärmetauscher (Apparat);
ii) direkte EK (direkte Energiekopplung), auch serielle EK genannt, die Verschaltung von mind. zwei baulich getrennten Wärmetauschern meint, unterteilt in:
   a. serielle integrierte EK, wobei ein Stoffstrom (integrierte stoffbasierte EK) oder Medienstrom (integrierte medienbasierte EK) aus einem ersten Wärmetauscher weitergeleitet wird zu einem stromabwärtsbefindlichen zweiten Wärmetauscher für die nächste integrierte EK, um jeweils auf einen (unterschiedlichen) Stoffstrom oder (unterschiedlichen) Medienstrom einzuwirken,
   b. serielle stoffbasierte EK (kurz "sEK"), wobei ein Stoffstrom aus einem ersten Wärmetauscher weitergeleitet wird zu einem stromabwärtsbefindlichen zweiten Wärmetauscher, um jeweils auf einen (unterschiedlichen) Medienstrom einzuwirken und/oder
   c. serielle medienbasierte EK (kurz "mEK"), wobei ein Medienstrom aus einem ersten Wärmetauscher weitergeleitet wird zu einem zweiten stromabwärtsbefindlichen Wärmetauscher, um jeweils auf einen (unterschiedlichen) Stoffstrom einzuwirken.

Im vorliegenden Zusammenhang meint EK allerdings keinen (üblichen) solitären Wärmetauscher ohne weitere (thermische) Verknüpfung, in dem von (nur) einem Wärmetauschmedium auch (nur) ein Edukt- oder Stoffstrom erwärmt oder abgekühlt wird. Weiterhin meint im vorliegenden Zusammenhang "weiterer Stoffstrom" einen Stoffstrom an einem anderen Anlagenort, in einer anderen stofflichen Zusammensetzung und/oder auf einem anderen Temperaturniveau und in analoger Weise ist "ein weiterer Medienstrom" zu verstehen.

Bei einer vorteilhaften Variante dieser Ausführungsform der Anlage kann vorgesehen sein, dass die Anlage (100) mind. eine serielle Verschaltung von zwei Wärmetauschern zur integrierte stoffbasierten EK, wobei in serieller Verschaltung ein Stoffstrom vom ersten Wärmetauscher in integrierter stoffbasierter EK zum zweiten stromabwärts befindlichen Wärmetauscher zur weiteren integrierten stoffbasierten EK weitergeleitet wird.

Bei einer weiteren vorteilhaften Ausführungsform der Anlage kann vorgesehen sein, dass mind. ein Wärmetauschkreislauf als direkte EK (serielle EK) ausgebildet ist, in den mind. zwei Wärmetauscher zur seriellen sEK oder mEK eingebunden sind. Vorteilhafterweise ist der Wärmetauschkreislauf ein geschlossener Kreislauf. Insgesamt kann es bei EK und Kreisläufen als EK vorteilhaft sein, bedarfsweise Wärmetauscher als Bedarfs- oder Regelungswärmetauscher mit insb. unabhängige Kühlmittel- oder Wärmequelle vorzusehen, um insgesamt die thermodynamische-energetische Steuerung und Regelung der Anlage sicherstellen zu können und einen zusätzlichen regelungstechnischen Freiheitsgrad zu erreichen. Diese Bedarfs- oder Regelungswärmetauscher sind insb. bei einer integrierten stoffbasierten EK in der Regel baulich deutlich kleiner, der vorherige, integrierte Wärmetauscher, der nach der integrierten stoffbasierten EK entfallen ist.

Bei einer weiteren vorteilhaften Ausführungsform der Anlage kann vorgesehen sein, dass mind. einer der nachfolgenden Leitungen im Kondensator in der (Kopf-)Leitung des Trennkessels als Wärmequelle zur integrierten stoffbasierten EK wie folgt eingebunden sind:
i) die (Feed-)Leitung zum Hauptreaktor
ii) die (Feed-)Leitung (116) zum Nachreaktor,
iii) die (Feed-)Leitung zur ersten Trennkolonne, wobei in der (Feed-)Leitung stromaufwärts zum Kondensator eine Druckregelungseinheit angeordnet ist,
iv) stromabwärts zu einer Einbindung einer Leitung nach i), ii) oder iii) im Kondensator und stromaufwärts zu einem Sammelbehälter mind. ein weiterer Wärmetauscher zur integrierten stoffbasierten EK eingebunden ist.

Bei einer weiteren vorteilhaften Ausführungsform der Anlage kann vorgesehen sein, dass die Anlage eine serielle mEK als Kreislauf mit einer Mehrzahl an Leitungsabschnitten umfasst, wobei mind. die folgenden Apparate und/oder Leitungen eingebunden sind: der Sammelbehälter stromabwärts zum Trennkessel, die (Kopf-)Leitung als ein Leitungsabschnitt, insb. ein erster Leitungsabschnitt, die (Feed-)Leitung zum Trennkessel als ein weiterer Leitungsabschnitt, insb. ein letzter Leitungsabschnitt. Bei dieser Ausführungsform wird der (Wärmetausch-)Kreislauf vom Lösungsmittel als Wärmetauschfluid durchflossen und der erste Strömungsweg des Hauptreaktors ist ein Leitungsabschnitt des Kreislaufes. Die Benennung von Leitungsabschnitten als erster oder letzter Leitungsabschnitt weisen auf eine Fließrichtung im Kreislauf hin, sollen aber ansonsten keine Einschränkung darstellen.

Bei einer Variante dieser Ausführungsform der Anlage kann vorgesehen sein, dass die (Feed-)Leitung zur ersten Trennkolonne der ersten Trennstufe im Kondensator in der (Kopf-)Leitung des Trennkessels zur integrierten stoffbasierten EK in den (Wärmetauscher-)Kreislauf eingebunden ist, wobei die (Kopf-)Leitung als Wärmequelle für die (Feed-)Leitung dient. Hierbei ist es besonders vorteilhaft, wenn in der (Feed-)Leitung stromaufwärts zum Kondensator eine Druckregelungseinheit angeordnet ist.

Bei einer weiteren vorteilhaften Ausführungsform der Anlage kann vorgesehen sein, dass als Wärmequelle der Medienumlauf des Hauptreaktors im Wärmetauscher mit der (Feed-)Leitung zur ersten Trennkolonne zur EK eingebunden sind. Hierbei ist in der (Sumpf-)Leitung stromaufwärts zum Wärmetauscher des Medienumlaufs eine Druckregelungseinheit angeordnet. Hierdurch liegt ein sehr vorteilhaftes Temperaturniveau in der (Feed-)Leitung zur ersten Trennkolonne vor, so dass ein stromabwärts eingebundener Wärmetauscher auf einem vorteilhaft niedrigen Temperaturniveau entsprechend große Wärmemengen aufnehmen kann.

Vorliegend wird häufig ein in Klammern gesetztes mehrteiliges Substantiv verwendet, wie bspw. (Feed-)Leitung, (Sumpf-)Leitung, (Kopf-)Leitung etc. Diese in Klammern gesetzten Substantive dienen der Veranschaulichung und leichteren sprachlichen Erfassung des Gemeinten, sollen aber kein Limitierung darstellen, da bspw. eine (Kopf-)Leitung eines Behälters, stromabwärts eine (Feed-)Leitung darstellt, die bspw. mit einen zugehörigen Behälter verbunden ist. Weiterhin werden "Strömungsweg", "Stoffstrom", "Medienstrom", "Eduktstrom" zum Teil als Synonym für die zugehörige Leitung verwendet, was insb. für die Beschreibungen der Anlagen. den zugehörigen Apparaten, Bauteilen und Elementen und deren Anordnung zueinander erfolgt ist. In analoger Weise meint eine EK immer auch einen entsprechenden Apparat, wie einen Wärmetauscher, Kondensator, etc..

Bei einer weiteren vorteilhaften Ausführungsform der Anlage kann vorgesehen sein, mind. eine der nachfolgenden Leitungen in einem Kondensator einer Trennkolonne der zweiten Trennstufe in der jeweiligen (Kopf-)Leitung als Wärmequelle zur integrierten stoffbasierten EK wie folgt einzubinden:
i) die (Feed-)Leitung zum Hauptreaktor,
ii) die (Feed-)Leitung zum Nachreaktor,
iii) die (Feed-)Leitung zur ersten Trennkolonne der ersten Trennstufe, wobei in der (Feed-)Leitung stromaufwärts zum Kondensator der jeweiligen Trennkolonne eine Druckregelungseinheit angeordnet ist.

Bei einer vorteilhaften Ausführungsform der Anlage kann vorgesehen sein, dass die Medienleitung:
i) vom Kondensator des Trennkessels der Trenneinheit als Wärmequelle zu mind. einem der folgenden Wärmetauscher der Reaktoreinheit oder der Konditionierungseinheit führt:
   - Wärmetauscher in einer (Feed-)Leitung zum Hauptreaktor und/oder einem stromaufwärts zum Hauptreaktor angeordneten Apparat, insb. Mischer oder Sättiger,
   - Wärmetauscher in der (Feed-)Leitung zum Nachreaktor und/oder
   - Wärmetauscher in der (Feed-)Leitung zur ersten Trennkolonne der Trenneinheit, und wobei mind. zwei der als direkte EK arbeitenden Wärmetauscher zueinander parallel geschaltet sein können;
ii) vom (kühlenden) Wärmetauscher des Reaktors als Wärmequelle zu mind. einem der folgenden Wärmetauscher führt:
   - Wärmetauscher in einer (Feed-)Leitung zum Hauptreaktor und/oder einem stromaufwärts zum Hauptreaktor angeordneten Apparat, insb. Mischer oder Sättiger,
   - Wärmetauscher in der (Feed-)Leitung zur ersten Trennkolonne der Trenneinheit, und wobei mind. zwei der als direkte EK arbeitenden Wärmetauscher zueinander parallel geschaltet sein können; und/oder ;
iii) von einem der (kühlenden) Wärmetauscher im Kopfumlauf eines Apparats der Trenneinheit zu mind. einem der nachfolgenden (heizenden) Wärmetauscher im Feed oder Sumpfumlauf eines Apparates der Trenneinheit führt, insb.
   - vom Wärmetauscher der Trennkolonnen der zweiten Trennstufe zum Wärmetauscher in der (Feed-)Leitung zur ersten Trennkolonne,
   - vom Kondensator im Kopfkreislauf der ersten Produkt abtrennenden Trennkolonne, insb. Produkt PACM, als Wärmequelle zum Wärmetauscher in der (Feed-)Leitung zur ersten Trennkolonne,.
   - vom Kondensator im Kopfkreislauf der Trennkolonne als Wärmequelle zum Wärmetauscher im Sumpfumlauf des Trennkessels.

In dem vorliegenden Zusammenhang meint "Medienleitung" eine Leitung, in der ein Wärmtauschfluid strömt, das weder Eduktmischung noch Stoffmischung ist. In der Regel ist das Wärmetauschfluid, Wasser, Wasserdampf, eine Sole oder ein Öl. In analoger Weise ist im vorliegenden Zusammenhang auch "Medienkreislauf" zu verstehen.,

Gemäß einer weiteren Ausführungsform der Anlage kann es vorteilhaft sein, wenn die Trenneinheit in der ersten Trennstufe in der (Feed-)Leitung zum Trennkessel mind. eine Druckregeleinheit und einen Trennkessel mit einer Kondensationseinheit für das Lösungsmittel umfasst. Hierbei kann vorteilhafterweise eine (Rück-)Leitung für das Lösungsmittel von der ersten Trennstufe, insb. einem Sammelbehälter, zur Konditionierungseinheit führen.

Der von dem Trennkessel kommende flüssige Stoffstrom wird über eine (Feed-)Leitung zur ersten Trennkolonne der ersten Trennstufe der Trenneinheit geleitet. Vorteilhafterweise kann bei einer Ausführungsform der Anlage vorgesehen sein, dass in der (Feed-)Leitung vom Trennkessel zur ersten Kolonne eine Druckregeleinheit vorgesehen ist. Hierdurch kann die Reaktoreinheit auf einem ersten, hohen Druckniveau betrieben werden und die erste Trennkolonne der erste Trennstufe auf einem zweiten, tieferen Druckniveau, wobei der Trennkessel auf einem Zwischenniveau betrieben werden kann.

Weiterhin kann es vorteilhaft sein, wenn stromaufwärts zur ersten Trennkolonne ein Wärmetauscher vorgesehen ist, insb. auch stromabwärts der Druckregeleinheit bzw. zwischen der Druckregeleinheit und der ersten Trennkolonne. Bei einer vorteilhaften Ausführungsform sind Medienleitungen vorgesehen, um in einer seriellen mEK den (Vorlauf-)Wärmetauscher des Nachreaktors oder dem Kondensator im Kopfkreislauf des Trennkessels mit dem (Vorlauf-)Wärmetauscher der ersten Trennkolonne zu verschalten.

Durch direkten Energieeintrag bspw. mittels überhitzten Dampfs, kann in einem ersten Schritt der vom Hauptreaktor kommende Stoffstroms (Feed der ersten Trennkolonne) um ca. 5 bis 20°C in einem (Vorlauf-)Wärmetauscher erwärmt werden, der nach der stromaufwärtsbefindlichen Druckregeleinheit ein eingangsseitiges Temperaturniveau von ca. 85 bis 95 °C aufweist. Das abströmende Heizmedium, ein Dampf-Kondensatgemisch, kann stromabwärts zu einem seriell gekoppelten (Vorlauf-)Wärmetaucher des Nachreaktors geleitet werden. Der Vorteil einer solchen Lösung besteht darin, dass die erste Trennkolonne weiterhin einen Sumpfumlauf und einen dort eingebundenen (Sumpf-)Wärmetauscher aufweist, der bedarfsweise den vollständigen Energiebedarf der ersten Trennkolonne decken kann. Somit kann durch die Kopplung der optimale Energietausch mit dem medienseitig stromabwärts befindlichen (Vorlauf-)Wärmetauscher (smEK) des Nachreaktors vorgenommen werden, während die verbleibende Energiemenge als Energieeinsparung der ersten Trennkolonne zufließen kann.

Bei einer besonders vorteilhaften Ausführungsform ist eine Energiekopplung vorgesehen, um den Kondensator stromabwärts zum Trennkessel, einen Kondensator einer Trennkolonne der zweiten Trennstufe oder den (Umlauf-)Wärmetauscher im Medienumlauf des mind. einen Hauptreaktors verschaltet im Wärmetausch mit dem (Vorlauf-)Wärmetauscher der ersten Trennkolonne zu betreiben. Die Energiekopplung kann durch Medienleitung und eine serielle Verschaltung der jeweiligen Wärmetauscher erfolgen oder durch eine integrierte Energiekopplung in einem (baulich) einzigen Wärmetauscher. Die integrierte Energiekopplung hat den Vorteil, sofern räumlich innerhalb der Anlage realisierbar, dass nur ein Temperaturgefälle für den Wärmetransport überwunden werden muss. Durch Energieübertrag kann so der (Feed-)Stoffstrom vor der ersten Trennkolonne der ersten Trennstufe, der nach der stromaufwärtsbefindlichen Druckregeleinheit in der (Feed-)Leitung ein Temperaturniveau von ca. 85 bis 95 °C aufweist, parallel angehoben werden.

Da der Hauptreaktor im Laufe des Betriebes bei stetig steigender Temperatur betrieben wird, um die sinkende Katalysatoraktivität zu kompensieren, kann parallel eine stetig steigende Energiemenge an den (Feed-)Stoffstrom vorlaufend der ersten Trennkolonne abgegeben werden. Hierdurch sinkt ebenfalls stetig der Energiebedarf im Sumpfkreislauf, bzw. dem dortig eingebundenen Wärmetauscher der ersten Trennkolonne.

Bei einer Ausführungsform der Anlage, kann es vorteilhaft sein, dass die Reaktoreinheit einen ersten Hauptreaktor und mind. einen stromabwärts befindlichen, in Serie geschalteten Nachreaktor umfasst. Der besondere Vorteil des Nachreaktors und dessen eingangsseitige Temperierung des Stoffstroms besteht darin, dass auf diese Weise eine optimierte Steuerung der Selektivität der Anteile an Isomeren ermöglicht wird, aufgrund der vom Hauptreaktor abweichenden, bevorzugt höheren Eintrittstemperatur.

Vorteilhafterweise ist der Nachreaktor auch ein Festbettreaktor, bzw. Reaktor mit einem immobilen Katalysator, wie beispielsweise einer Katalysatorschüttung oder mit Katalysator beschichteten Einbauten.

Vorteilhafterweise umfasst der immobile Katalysator Ruthenium, ist mit Ruthenium dotiert oder ist hieraus gebildet. Bei einer vorteilhaften Verfahrensvariante, insb. zur Erreichung eines niedrigen Anteils an trans/trans Isomeren im Isomerengemisch, wird der Hauptreaktor bei einer Temperatur von 90 bis 140 °C, idealerweise 95 bis 135 °C.

Es hat sich als besonders vorteilhaft herausgestellt, wenn das Verhältnis der Katalysatormassen vom Hauptreaktor zum Nachreaktor ist im Bereich von 1,2 bis 2 liegt, bevorzugt 1,3 zu 1,4 und idealerweise 1,35. Es hat sich überraschenderweise gezeigt, dass es hinreichend ist, die stark exotherme Reaktion beim den Reaktionsstart im Hauptreaktor durch einen intensiven Kühlkreislauf zu regeln, und lediglich die Vorlauftemperatur im Zulauf zum Nachreaktor so einzustellen, dass der moderate Temperaturanstieg von ca. 30 bis 35 °C im Nachreaktor vom Einlass zum Auslass nur noch einen limitierten und gut reproduzierbaren Einfluss auf den trans/trans-Isomerenanteil im Stoffstrom bzw. im Produkt hat, wie bereits ausgeführt.

Es kann insb. vorteilhaft sein, wenn der Hauptreaktor ein Festbettreaktor ist, der
- einen ersten Strömungsweg für das Edukt- bzw. Stoffgemisch und
- einen weiteren (geschlossenen) Strömungsweg, d.h. einem Medienumlauf für ein Wärmetauschmittel umfasst, wobei in den zweiten Strömungsweg zwei Wärmetauscher für einen indirekten Wärmeaustausch eingebunden sind:
   - einen als Kühler arbeitenden (Haupt-)Wärmetauscher und
   - einen als Heizer arbeitenden (Neben-)Wärmetauscher.

Es hat sich als sehr effektiv und vorteilhaft herausgestellt, denselben weiteren Strömungsweg, bzw. Medienumlauf zum Vorbereiten und Anfahren des Hauptreaktors mittels des (Neben-)Wärmetauschers zu betreiben und die Kühlung der Hauptreaktion im produzierenden Betrieb des Hauptreaktors mittels des (Haupt-)Wärmetauschers zu betreiben.

Bei einer weiteren Ausführungsform der Anlage, kann es vorteilhaft sein, dass die Reaktoreinheit einen weiteren Hauptreaktor als Festbettreaktor umfasst, der
- einen ersten Strömungsweg für das Stoffgemisch und
- einen weiteren (geschlossenen) Strömungsweg, d.h. einem.(Kühl-)Medienumlauf für ein Wärmetauschmittel umfasst, und wobei stromaufwärts zu den beiden Hauptreaktoren in der (Zu-)Leitung eine Ventileinheit vorgesehen ist, mittels welcher der Volumenstrom der Eduktmischung zw. dem ersten Hauptreaktor und dem weiteren Hauptreaktor aufteilbar, durchleitbar und/oder vollständig umschaltbar ist, und wobei beide Hauptreaktoren
- jeweils mit einem Wärmetauscher oder
- einem gemeinsam mit einem Wärmetauscher
für den weiteren (geschlossenen) Strömungsweg (wärmeleitend) verbunden sind.

Hierbei sind die beiden in Reihe geschalteten Hauptreaktoren baugleich oder im Wesentlichen baugleich. Insbesondere sind beide Hauptreaktoren derart dimensioniert und/oder weisen entsprechende Einbauten auf, dass dieselbe bzw. die im Wesentliche gleiche Masse und/oder Volumen an Katalysator angeordnet ist oder aufnehmbar ist.

Vorteil dieser beiden Hauptreaktoren liegt darin, dass eine weitere thermische Entkopplung der ersten Reaktionsphase mit sehr starker Exothermie, der zweite Reaktionsphase mit mittlerer Exothermie und der Nachreaktion mit sehr geringer Exothermie erfolgen kann. Weitere Vorteile bestehen darin, dass bei derselben Anlagenleistung, der einzelne Hauptreaktor kleiner dimensioniert ist und somit thermisch leichter und homogener geregelt werden kann. Zeitgleich wird die Anlageleistung erhöht, weil im Wartungsfall, wie bspw. einem Katalysatorwechsel, die Anlage nicht vollständig stillgestellt werden muss. Hierzu sind die beiden Hauptreaktoren derart leitungstechnisch verschaltet, dass jeder auch alleinig vom Stoffgemisch durchströmbar ist, unter Umgehung des jeweils andere Hauptreaktors (Bypass 1).

Durch die Hydrierung im (isothermischen) Hauptreaktor mit starker Kühlung, kann die durch die Temperatur induzierte Isomerisierung stark begrenzt werden. Im (adiabatischen) Nachreaktor wird anschließend gezielt so viel Temperatur insb. durch Wärmetausch im zufließenden Stoffstrom eingeregelt und damit zugelassen, dass die Isomerisierung gerade einen spezifikationsgerechten trans/trans-Anteil im Produkt liefert. Bei einer rein isothermen Betriebsweise eines einzelnen Hauptreaktors ohne Nachreaktor, würde man zunächst zu niedrige trans/trans-Anteile und einen hohen Anteil an nicht umgesetztem MDA erhalten. Durch die Möglichkeit den Stoffstrom gezielt adiabatisch hydrieren zu lassen, kann die Temperaturentwicklung und damit die Isomerisierung im Nachreaktor vorteilhaft gesteuert werden.

Bei einer weiter verbesserten Variante ist die leitungstechnische Verschaltung derart, dass auch der Nachreaktor beim Betrieb von mindestens einem Hauptreaktor umgangen entweder kann (Bypass 2), insb. aber beim Betrieb von den beiden in Reihe geschalteten Hauptreaktoren umgangen werden kann. Bei der Verschaltungsvariante Bypass 2, übernimmt der in Strömungsrichtung als zweiter durchflossene Hauptreaktor mind. zeitweise die Funktion des Nachreaktors, so dass die Anlage ohne bzw. weitgehend ohne Leistungsabfall und/oder Änderungen der Produktqualität, insb. des jeweiligen Anteils an Isomeren im Isomerengemisch betrieben werden kann.

Bei einer weiteren Ausführungsform der Anlage, kann es vorteilhaft sein, dass in der Leitung zwischen dem mind. einen Hauptreaktor und dem zweiten Hauptreaktor mind. ein gemeinsamer Wärmetauscher angeordnet ist. Dieser gemeinsame Wärmetauscher ist in einem zentralen Ast beider Kühlmittelumläufe, angeordnet. Leitungsführung und Leitungsverschaltung ist dabei derart, dass nach dem gemeinsamen Wärmetauscher das Kühlmedium zuerst in den ersten der beiden Hauptreaktoren eingeleitet wird, in dem die stärker exotherme Reaktion abläuft. Anschließend wird über eine Leitung das bereits erwärmte Kühlmedium in den stromabwärts befindlichen zweiten Hauptreaktor geleitet, so dass dieser mit einer vom ersten Hauptreaktor unterschiedlichen Vorlauftemperatur beschickt wird.

Der Vorteil besteht darin, dass es überraschenderweise beobachtet wurde, dass insb. die sehr genaue Regelung der ersten, stark exothermen Reaktionsphase für die Produktqualität entscheidend ist, so dass auf den baulichen und regelungstechnischen Aufwand eines weiteren, vollständig unabhängigen zweiten Kühlkreislaufs verzichtet werden kann. Dies insb. auch deshalb, weil über die Regelung der Vorlauftemperatur des zu den beiden Hauptreaktoren seriell nachgeschalteten Nachreaktors die vollständige Reaktion sichergestellt und geregelt werden kann, insb. der gewünschte, niedrige trans/trans-Isomerenanteil eingeregelt werden kann.

Bei einer Anlagenvariante mit einer verbesserten Regelbarkeit kann vorgesehen werden, dass in der jeweiligen (Kreuz-)Leitung der Kühlkreisläufe, mit denen der Kühlmittelauslass des ersten Hauptreaktors mit dem Kühlmitteleinlass des zweiten Hauptreaktors verbunden ist, ein bedarfsweise schaltbarer und regelbarer Wärmetauscher (Nachkühler) angeordnet ist.

Der Sumpfumlauf des Trennkessels wird bei einer Temperatur von 130 °C bis 150 °C, idealerweise 135 bis 145 °C betrieben. Der große Vorteil des baulich und regelungstechnisch sehr einfachen Trennkessels ist, dass die Siedetemperatur des Gemisches durch die Druckabsenkung ebenfalls gesenkt wird, so dass nur auf diese erniedrigte Siedetemperatur im Trennkessel aufgeheizt werden muss. Weiterhin sind bereits durch diese Maßnahme ca. 90 % des in der Eduktmischung vorliegenden Lösungsmittels, insb. THF, abtrennbar. Ein Kopfkreislauf oder Rücklaufstrom, wie bei einer Kolonne, ist hierzu nicht erforderlich. Der zur ersten Trennkolonne weitergeleitete Stoffstrom weist somit vorteilhafterweise nur eine verbleibende Lösungsmittelkonzentration von ca. 20 bis 40 Gew.% auf, idealerweise 25 bis 35 Gew.%. Somit kann die erste Trennkolonne baulich kleiner ausfallen und ist aufgrund der geringeren Masse des Stoffstroms energetisch sparsamer betreibbar.

Auf diese Weise ist die Temperatur des Stoffstrom zudem schneller absenkbar, so dass im Stoffstrom ein Anstieg des Anteils an trans/trans-Isomeren des Produkts, insb. des Produkts PACM, verhindert bzw. verringert wird.

Die Lösungsmittelführende (Rück-)Leitung ist der Konditionierungseinheit und/oder mind. einem geeigneten Sammeltank verbunden.

Schließlich kann eine vorteilhafte Ausführungsform der Anlage darin bestehen, dass mind. ein Wärmetauschkreislauf als serielle integrierte stoffbasierte EK umfasst ist, der eine Mehrzahl von Leitungsabschnitten aufweist, in den mind. die folgenden Apparate und Wärmetauscher eingebunden und über mind. einen der Leitungsabschnitte verbunden sind,
i) als Wärmetauscher und als Wärmequelle für den Wärmetauschkreislauf
   - der Wärmetauscher im Medienumlauf des Hauptreaktors **oder**
   - der (Kopf-)Wärmetauscher in der (Kopf-)Leitung des Trennkessels, jeweils in integrierter stoffbasierter EK mit mind. einer der nachstehenden Leitungen mit dem darin geführten Edukt- oder Stoffstrom als Wärmesenke für den Wärmetauschkreislauf:
      a) der (Feed-)Leitung zur ersten Trennkolonne, stromabwärts zur Druckregeleinheit,
      b) der (Feed-)Leitung zum Nachreaktor, insb. auf der Saugseite einer dort eingebundenen Pumpe,
      c) der (Feed-)Leitung stromaufwärts zum Reaktor, insb. einem hierzu stromaufwärts befindlichen Mischbehälter, oder der (Rück-)Leitung zur Konditionierungseinheit stromaufwärts zum Mischer;
ii) als Apparate mind. der Hauptreaktor, insb. der Hauptreaktor und der Nachreaktor, mind. ein Trennkessel, mind. ein Sammelbehälter der ersten Trennstufe, und wobei
iii) die folgenden Leitungen als Leitungsabschnitte des Kreislaufs integriert sind:
   a) ein (der erste) Leitungsabschnitt, welcher der (Kopf-)Leitung des mind. einen Trennkessels entspricht und/oder
   b) ein (letzter) Leitungsabschnitt, welcher der (Feed-)Leitung zu dem mind. einen Sammeltank der zweiten Trennstufe entspricht.

Von der Erfindung ist weiterhin ein Verfahren umfasst zur kontinuierlichen, katalytischen Hydrierung von Methylendianilin (MDA; Edukt1), mit einem Wasserstoffgeber (Edukt2), insb. einem gasförmigen Wasserstoffgeber, vorzugsweises Wasserstoff (H2), wobei die Herstellung mittels einer industriellen Anlage erfolgt, wobei die Anlage nach mindestens einem der hierin beschriebenen Ausführungsbeispielen und Varianten ausgebildet ist, und wobei der Hauptreaktor bei einer Temperatur im Bereich von 80 °C bis 150 °C betrieben wird.

Bei einer vorteilhaften Ausführungsform wird durch Energiekopplung (EK):
i) vom Kondensator der Trenneinheit, hier der Kondensator im Kopfkreislauf des Trennkessels, als Wärmequelle für mind. einen Wärmetauscher der Reaktoreinheit, der Konditionierungseinheit und/oder der Trenneinheit durch integrierte EK oder direkte EK Energie im Bereich von der verfügbaren Wärmemenge transferiert, insb. im Bereich von 20 bis 40 %;
ii) Wärmetauscher des (kühlenden) Medienumlaufs für den Hauptreaktor der Reaktoreinheit als Wärmequelle für mind. einen Wärmetauscher der Reaktoreinheit, der Konditionierungseinheit und/oder der Trenneinheit durch integrierte EK oder direkte EK Energie im Bereich von 5 bis 30 % der verfügbaren Wärmemenge transferiert wird, insb. im Bereich von 10 bis 20 % und/oder iii) Wärmetauscher der Trenneinheit untereinander durch integrierte EK oder direkte EK Energie im Bereich von 5 bis 100 % der verfügbaren Wärmemenge transferiert wird, insb. im Bereich von 30 bis 90 %.

Bei einer weiteren Ausführungsform des Verfahrens kann ein Vorteil darin bestehen, dass neben dem mind. einen Hauptreaktor weiterhin mind. ein Nachreaktor umfassend einen immobilen Katalysator vorgesehen ist, wobei der mind. eine Hauptreaktor und der mind. eine Nachreaktor bei demselben oder im Wesentlichen bei demselben Druck betrieben werden.

Bei einer weiteren Ausführungsform des Verfahrens kann ein Vorteil darin bestehen, dass die Temperatur des Eduktstroms am Einlass des Hauptreaktors 90 bis 140°C beträgt, idealerweise 100 bis 135 °C.

Bei einer bevorzugten Ausführungsform des Verfahrens kann vorgesehen werden, dass eine kontinuierliche, katalytische Produktion von Methylenbis(cyclohexylamin) erfolgt, insb. die Produktion von 4,4'-Diaminodicyclohexylmethan (PACM), bevorzugt 4,4'-Diaminodicyclohexylmethan (PACM) mit niedrigen Anteilen an trans/trans-Isomeren. Bei einer bevorzugten Verfahrensvariante erfolgt eine kontinuierliche, katalytische Herstellung von Methylenbis(cyclohexylamin), insb. eine Produktion von PACM, nach der Formel (I)

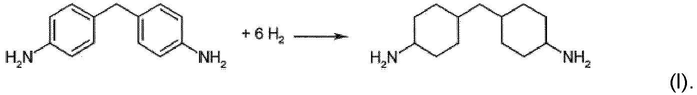

Bei einer weiteren Ausführungsform des Verfahrens kann ein weiterer Vorteil darin bestehen, dass der mind. eine Hauptreaktor bei einem Druck im Bereich von 60 bar bis 120 bar betrieben wird, idealerweise im Bereich von 70 bis 100 bar. Bei einer weiteren vorteilhaften Verfahrensführung kann vorgesehen werden, dass der Druck im Hauptreaktor 80 bis 90 bar beträgt. Besonders bevorzugt ist eine Druck von ca. 85 bis 95 bar.

Bei einer weiteren Ausführungsform des Verfahrens kann ein weiterer Vorteil darin bestehen, dass vom Zeitpunkt t₀, dem Verfahrensstart nach Erneuerung oder Regeneration des Katalysators bis zum Zeitpunkt t₄, dem Ende des Verfahrens, bestimmt durch die Erneuerung oder Regeneration des Katalysators, die Betriebstemperatur des Hauptreaktors erhöht und Temperatur im Stoffstrom im Einlass (Feed) des Nachreaktors gleichgehalten oder gesenkt wird, wobei die Temperaturerhöhung oder -Absenkung linear und/oder schrittweise erfolgt.

Mit der genannten Anlagenvariante ist somit ein Verfahren/-sablauf zu einer Erreichung eines niedrigen trans/trans-Anteils im PACM von 17 bis 25 Gew.% über der Zeit möglich.

Bei einer weiteren Ausführungsform des Verfahrens kann ein weiterer Vorteil darin bestehen, dass das MDA (Edukt1) eine Mischung der mind. zwei Isomere umfasst: 4,4' MDA, 2,4' MDA und 2,2' MDA. Das MDA (Edukt1) ist vorteilhafterweise eine Mischung, wobei die Mischung die folgenden Monomere umfasst: 4,4' MDA, 2,4' MDA und 2,2' MDA, wobei der Anteil an 4,4' MDA vorteilhafterweise im Bereich von 75 bis 98 mol-% liegt, bevorzugt 85 bis 95 mol-%, idealerweise ca. 90 mol-%. Der Anteil an 2,4' MDA im Eduktgemisch beträgt vorteilhafterweise 7 bis 15 mol-%, bevorzugt 8 bis 12 mol-%, idealerweise 9 bis 10 mol-%.

Idealerweise ist der Anteil an 4,4` PACM (trans/trans PACM) im Bereich von 15 bis 30 Gew.%, idealerweise 16 bis 25 Gew.%

Insgesamt ist es vorteilhaft, wenn der Reaktionsschritt im Hauptreaktor hierbei isotherm oder weitgehend isotherm und der Reaktionsschritt im Nachreaktor adiabat oder weitgehend adiabat betrieben wird. Zum Zeitpunkt t₀, dem Verfahrensstart kann neben dem Hauptreaktor auch der Katalysator im Nachreaktor erneuert oder regeneriert vorliegen. Vorteilhafterweise wird der Zyklus zur Regeneration des Nachreaktors eigenständig und vom Hauptreaktor unabhängig bestimmt, insb. wenn o.g. Zusammenwirken der Reaktionsanteile in beiden Reaktoren nicht mehr die Produktion des gewünschten niedrigen trans/trans-Anteils im PACM ermöglicht.

Bei einer vorteilhaften Ausführungsform des Verfahrens kann vorgesehen sein, dass die Temperatur des Eduktstroms am Einlass des Hauptreaktors 90 bis 140°C beträgt, idealerweise 100 bis 135 °C, vorzuzugsweise 105 bis 115 °C.

Bei einer weiteren vorteilhaften Ausführungsform des Verfahrens kann vorgesehen sein, dass der Druck im Hauptreaktor 70 bis 100 bar beträgt, idealerweise 80 bis 90 bar.

Bei einer weiteren vorteilhaften Ausführungsform des Verfahrens kann vorgesehen sein, dass die Temperatur am Eingang des Hauptreaktors im Wesentlichen der Temperatur am Eingang des Nachreaktors entspricht, wobei im Wesentlichen einen Bereich oder Unterschied von +/- 10 °C meint und/oder der Druck am Eingang des Hauptreaktors im Wesentlichen dem Druck am Eingang des Nachreaktors entspricht, wobei im Wesentlichen einen Bereich oder Unterschied von +/- 5 bar meint.

Insgesamt sollen alle Aspekte, Vorteile und Ausführungen zur Anlagen oder in Zusammenhang mit deren Beschreibung genannt sind, identisch oder in analoger Weise auch für das Verfahren gelten und umgekehrt, sofern nicht etwas Abweichendes dargelegt ist und/oder eine technische Unmöglichkeit der analogen Anwendung gegeben ist.

Nachfolgend wird die erfindungsgemäße Lösung anhand von Ausführungsbeispielen im Detail beschrieben. Es zeigen:
- Fig. 1: eine Anlage als Prozessfließbild,
- Fig. 2: eine erste Ausführungsform der Anlage mit einer ersten direkten EK,
- Fig. 3: eine zweite Ausführungsform der Anlage mit einer weiteren direkten EK,
- Fig. 4: eine erste Ausführungsform der Reaktoreinheit,
- Fig. 5: eine zweite Ausführungsform der Reaktoreinheit,
- Fig. 6 (ehem Fig.7): eine dritte Ausführungsform der Anlage mit einer weiteren direkten EK als integrierte stoffbasierte EK,
- Fig. 7 (ehem. Fig.6): eine vierte Ausführungsform der Anlage mit einer weiteren direkten EK als integrierte stoffbasierte EK,
- Fig. 8 (NEU): eine fünfte Ausführungsform der Anlage mit einer weiteren direkten EK als integrierte stoffbasierte EK,
- Fig. 9: eine sechste Ausführungsform der Anlage mit einer weiteren direkten EK als integrierte stoffbasierte EK mit serieller Verschaltung und Kreislaufführung,
- Fig. 10: eine siebte Ausführungsform der Anlage mit einem Kreislauf zur Medienweiterleitung und
- Fig. 11: eine achte Ausführungsform der Anlage mit einem zur Figur 10 alternativen Kreislauf zur Medienweiterleitung.

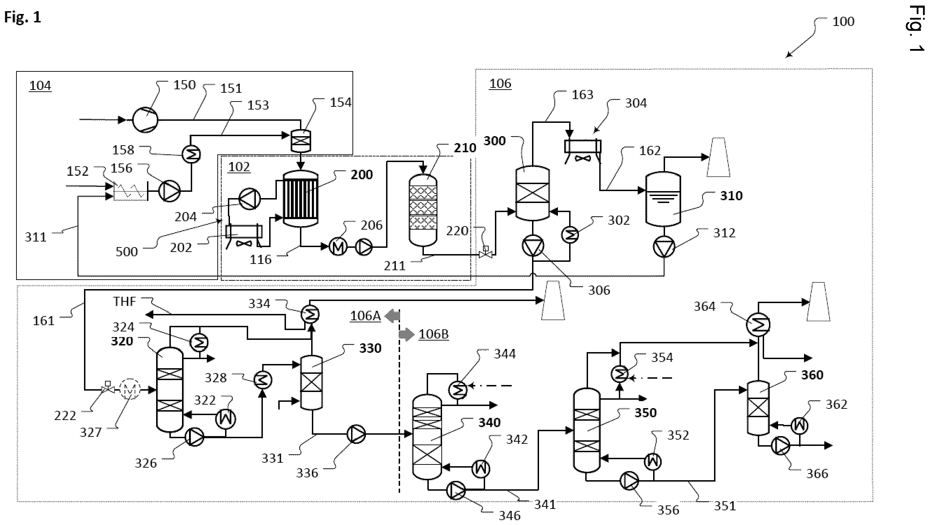

Die Figur 1 zeigt die Anlage 100 zur kontinuierlichen Herstellung von 4,4'-Diaminodicyclohexylmethan (PACM) durch katalytische Hydrierung von Methylendianilin (MDA; Edukt1), insb. 4,4`-Diaminodiphenylmethan, mit einem Wasserstoffgeber (Edukt2), wobei der Wasserstoffgeber als gasförmiger Wasserstoff (H2) zugeführt wird. Die Anlage 100 umfasst eine Konditionierungseinheit 104 für die Edukte, eine Reaktoreinheit 102 und eine Trenneinheit 106.

Die Konditionierungseinheit 104 ist gestrichelt umrahmt und umfasst (Zu-)Leitungen für das Edukt1 und den Wasserstoff (Edukt2) sowie das Lösungsmittel. Weiterhin umfasst die Konditionierungseinheit eine Verdichtereinheit 150, nachfolgend Verdichter genannt in der den Wasserstoff zuleitenden Leitung 151, einen Mischer 152 in der das Lösungsmittel und das Edukt1 zuführenden Leitung. Zudem sind in der die Mischung aus Edukt1 und Lösungsmittel zuführenden Leitung 153 eine Pumpe 156 und ein Wärmetauscher 158 angeordnet. Die Leitungen 151, 152 münden in einen Mischbehälter 154, der stromaufwärts zum Hauptreaktor 200 angeordnet ist. Der Mischbehälter 154 dient der intensiven Vermischung der Edukte und dessen Auslauf bildet den Zulauf für den Hauptreaktor 200.

Die Reaktoreinheit 102 ist gestrichelt umrahmt und umfasst im Wesentlichen den Hauptreaktor 200, einen Kühlumlauf 500, eine Nachreaktor 210 und einen Wärmetauscher 206 in der Zulaufleitung zum Nachreaktor 210. In den Kühlumlauf 500 ist eine Pumpe 204 und ein Wärmetauscher 202 eingebunden, wobei das umlaufende Kühlmittel im Hauptreaktor 200 die mit Katalysatormaterial befüllten Trägerelemente umströmt. Im gezeigten Beispiel werden die mit Katalysatormaterial befüllten Trägerelemente im Gleichstromrichtung angeströmt. Der Nachreaktor 210 ist über die Leitung 211 mit dem Trenneinheit 106, bzw. deren ersten Trennstufe verbunden.

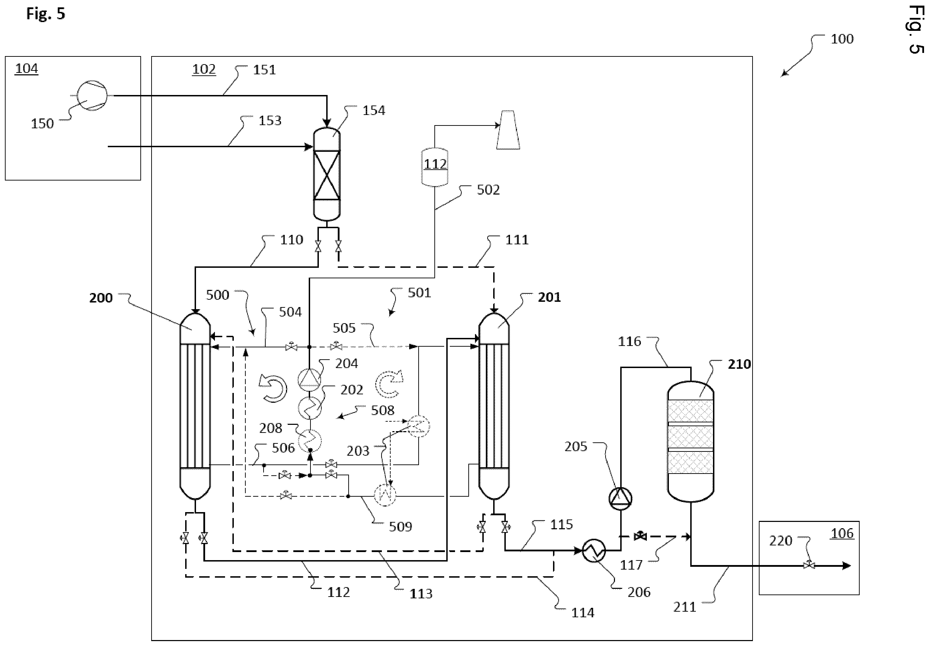

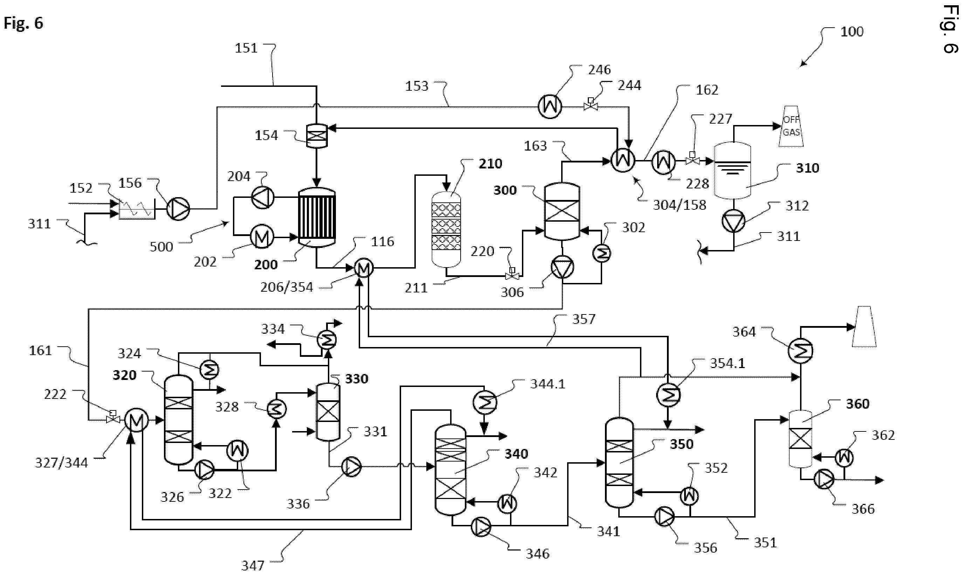

Der Wärmetauscher 202 im Kühlumlauf 500 ist als luftgekühlter Wärmetauscher 202 dargestellt, kann aber auch alternativ ausgebildet sein, um bspw. mittels eines fließenden Kühlmediums, wie bspw. ein Öl, Wasser oder eine Sole, um im indirekten Wärmeaustausch das Kühlmedium des Kühlumlaufs 500 zu temperieren. In den Kühlumlauf 500 ist weiterhin bei einer nichtdargestellten Anlagenvariante ein Wärmetauscher eingebunden, analog den Wärmetauschern 208, 209 der Figuren 5, 6. Dieser wird mit einem Heizmedium betrieben und dient im Anfahrschritt des Hauptreaktors 200 zur Temperierung des Hauptreaktors 200 auf ca. 80 bis 100 °C, idealerweise auf eine Temperatur von 85 °C bis 95 °C. In dem gezeigten Anlagen- und Verfahrensbeispiel, bei dem Ziel ein möglichst niedriges trans/trans-Isomerenverhältnis von ca. 17 bis 23 Gew.% zur realisieren, wird der mit frischem oder regeneriertem Katalysator befüllte Hauptreaktor 200 auf eine Temperatur von ca. 90 °C mittels des Wärmetauschers 208 vorgeheizt. Der Hauptreaktor 200 wird bei einem Druck von 87 bis 88 bar betrieben.

Die Trenneinheit 106 ist gestrichelt umrahmt und umfasst eine Mehrzahl von Trennapparaten zur Abtrennung des Lösungsmittel insb. in einer ersten Trennstufe und des Produkts PACM, insb. in einer zweiten Trennstufe, von restlichem Edukt und Nebenprodukten. Die erste Trennstufe (nicht ausgewiesen) umfasst einen Trennkessel 300 an den ein Sumpfumlauf angeschlossen ist, in den ein Wärmetauscher 302 und eine Pumpe 306 eingebunden ist. Der Kopfauslass des Trennkessels 300 ist mit einem Wärmetauscher 304, einem Kondensator, verbunden, zu dem stromabwärts ein Sammelbehälter 310 für das Lösungsmittel vorgesehen ist. Mittels des Wärmetauschers 304 (Kondensator) werden ca. 80% des Lösungsmittels, vorliegend THF, auskondensiert und könnten gesammelt bzw. zurückgeleitet werden. In der Flash-Stufe ist ein Energiebedarf für den Wärmetauscher 302 im Sumpfumlauf des Trennkessels 300 von ca. 1400 kW erforderlich.

Der Kondensator 304 ist als Wärmetauscher in der Bauform eines luftgekühlten Apparates dargestellt, kann aber auch alternativ ausgebildet sein, um bspw. mittels eines fließenden Kühlmediums, wie bspw. Kühlwasser oder einem zur Wärmeintegration geeigneten Medium, um im indirekten Wärmeaustausch den eingangs dampfförmigen Stoffstrom mind. teilweise zu kondensieren und zu kühlen.

Die hierin genannten Energiemengen sind kalkuliert für eine Anlagenleistung des Produktes PACM bei der genannten Synthesereaktion von ca. 3,37 t/h, sowie eine Nebenproduktleistung von ca. 0,4 t/h an HB und ca. 0,05 t/h an LB. Das Verhältnis der Massenströme von THF zu MDA betrugt 4,2 bis 4,5.

Weiterhin umfasst die erste Trennstufe der Trenneinheit 106 zur weitergehenden Abtrennung des Lösungsmittels einen erste Trennkolonne 320 und eine zweite Trennkolonne 330, wobei die zweite Trennkolonne 330 als Stripping Kolonne ausgebildet ist. Hierzu wird vorteilhafterweise Stickstoff (N2) als Stripping-Medium eingesetzt, das im Gegenstrom zum Stoffstrom durch die Kolonne geleitet wird. Mittels der im Sumpfauslass des Trennkessels 300 befindlichen Pumpe 306 ist der an Lösungsmittel verarmte Stoffstrom über die Leitung 161 zur ersten Trennkolonne 320 ableitbar. In der Leitung 161 ist eine Druckregeleinheit 222 vorgesehen, die im gezeigten Beispiel als regelbares Ventil ausgebildet ist. Der ersten Trennkolonne 320 wird der Stoffstrom über einen zentralen Zulauf wie dargestellt eingeleitet. Weiterhin ist stromaufwärts zur ersten Trennkolonne 320 als optionale Ausführungsform ein (Vorlauf-)Wärmetauscher 327 (gestrichelt dargestellt) angeordnet, der eine Option zur Beheizung der ersten Trennkolonne 320 darstellt.

In der ersten Trennkolonne 320 wird die Lösungsmittelkonzentration von 30 Gew.% im Zulauf über den produktreichen Stoffstrom der Leitung 161 auf ca. 2 Gew.% an restlichem Lösungsmittel verringert.

Diese erste, mit (Struktur-)Packungen bestückte, Trennkolonne 320 ist an einen Sumpfumlauf angeschlossen, in den ein Wärmetauscher 322 und eine Pumpe 326 eingebunden sind. Weiterhin ist am Kolonnenkopf der ersten Trennkolonne 320 ein Kopfumlauf angeordnet, in den ein Wärmetauscher 324 eingebunden ist, der als Kondensator ausgebildet ist. Aus dem Kopfumlauf führen zwei Ableitungen für den lösungsmittelreichen Stoffstrom, wobei eine der beiden Ableitungen in die Kopfableitung der stromabwärts befindlichen Trennkolonne 330 (Stripping-Kolonne) einmündet.

Insbesondere ist das in der ersten Trennstufe abgetrennte Lösungsmittel über die Leitung 311 in einen Sammeltank (nicht dargestellt) und/oder den Mischer 152 der Konditionierungseinheit 104 leitbar. In der vom Nachreaktor 210 zum Trennkessel 300 führenden Leitung 211 ist eine Druckregeleinheit 220 vorgehen, die im vorliegenden Beispiel als steuerbares Ventil ausgebildet ist.

Über die Leitung 321 wird aus dem Sumpfauslass der ersten Trennkolonne 320 der produktreiche Stoffstrom mit einem Restanteil von ca. 2 Gew.% Lösungsmittel (THF) in den Kopf der zweiten Kolonne 330, der Stripping-Kolonne, geleitet. In dieser Leitung 321 ist ein Wärmetauscher 328 eingebunden. Die zweite Kolonne 330 weist mind. eine innere Packung auf, wobei unterhalb der Packung eine Gaszuleitung für insb. ein Inertgas (Strippinggas) angeordnet ist, so dass der eingeleitete produktreiche Stoffstrom im Gegenstromprinzip zum Strippinggas die zweite Kolonne 330 durchströmt und von Lösungsmittel weiter abgereichert wird. In dem gezeigten Anlagenbeispiel wird Stickstoff (N2) als Strippinggas eingesetzt. Der lösungsmittelreiche Dampf wird über den Kopfauslass in einen Kondensator 334 geleitet und gemeinsam mit dem von Kopfauslass der ersten Trennkolonne 320 kommenden und die Kopfableitung der zweiten Trennkolonne 330 eingeleitete Dampfstrom dem Kondensator 334 zugeleitet. Der im Wärmetauscher 334 auskondensierte Lösungsmittelstrom wird zur Konditionierungseinheit 104 zurückgeleitet, wobei der nicht-kondensierbare Anteil aus dem Wärmetauscher 334 abgeleitet und bspw. vollständig thermisch oxidiert wird.

Vom Sumpfauslass der zweiten Trennkolonne 330 wird der produktreiche Stoffstrom über die Leitung 331, in die die Pumpe 336 eingebunden ist, der dritten Trennkolonne 340 zugeführt. Die zweite Trennstufe 106B der Trenneinheit 106 dient insb. der Abtrennung der Nebenprodukte LB und/oder HB vom Produkt PACM.

Die zweite Trennstufe (106B) umfasst im Wesentlichen drei Trennkolonnen, wobei der dritten Trennkolonne 340, die erste der zweiten Trennstufe, der Stoffstrom zentral zugeführt wird. Die dritte Trennkolonne ist mit einem Sumpfumlauf verbunden, in den ein Wärmetauscher 342 und eine Pumpe 346 eingebunden sind. Der produktreiche Stoffstrom wird über die Leitung 341 aus dem Sumpfablauf zur vierten Trennkolonne 350 geleitet. Weiterhin ist die dritte Trennkolonne 340 mit einem Kopfumlauf verbunden, in den ein Wärmetauscher 344 eingebunden ist. Aus diesem Kopfumlauf wird auskondensiertes LB als erstes Nebenprodukt abgeleitet.

Der produktreiche Stoffstrom wird über die Leitung 341 der vierten Trennkolonne 350 zentral zugeführt. Die vierte Trennkolonne 350 ist mit einem Sumpfumlauf verbunden, in den ein Wärmetauscher 352 und eine Pumpe 356 eingebunden sind. Weiterhin ist die vierte Trennkolonne 350 mit einem Kopfumlauf verbunden, in den ein als Kondensator ausgebildeter Wärmetauscher 354 eingebunden ist. Der produktreiche Stoffstrom wird über die Kopfableitung aus dem Kopfumlauf als Kondensat ausgeleitet. Vorliegend wird gemäß dem gezeigten Beispiel, der nichtkondensierte Dampf- und/oder Gasstrom in die Kopfausleitung der stromabwärtsbefindlichen fünften Trennkolonne 360 eingeleitet. Nachfolgend wird über einen weiteren Wärmetauscher 364 (Kondensator) Produkt weiter auskondensiert und ausgeleitet. Der produktarme Stoffstrom wird über die Sumpfableitung der vierten Trennkolonne 350 über die Leitung 351 abgeleitet und der fünften Trennkolonne 360 in deren Kopfteil eingeleitet. Dieser Stoffstrom ist stark angereichert an HB, einem zweiten Nebenprodukt. Die fünfte Trennkolonne 360 ist mit einem Sumpfumlauf verbunden, in den ein Wärmetauscher 362 und eine Pumpe 366 eingebunden sind. Weiterhin weist die Trennkolonne 360 einen Kopfauslass auf, der zu dem vorgenannten als Kondensator ausgebildeten Wärmetauscher 364 führt. In diesem Kondensator 364 wird ein weiterer produktreicher Stoffstrom als Kondensat auskondensiert und ausgeleitet, wobei der nicht-kondensierbare Anteil gasförmig ausgeleitet wird. Dieser kann nachfolgend insb. vollständig oxidiert werden.

Die Reaktoreinheit 102 wird bzgl. des Stoffstroms auf einem ersten, hohen Druckniveau von ca. 60 bis 120 bar betrieben, die erste Trennstufe 106A der Trenneinheit 106 wird bei einem zweiten, niedrigen Druckniveau von 4 bis 12 bar betrieben und die erste Trennkolonne 320 und die zweite Trennkolonne 330 werden auf einem dritten, geringfügig erhöhten Druckniveau von 1,05 bis 2,5 bar betrieben. In dem gezeigten Beispiel ist das erste Druckniveau 80 bis 90 bar, das zweite Druckniveau 4,5 bis 7,5 bar und das dritte Druckniveau 1,1 bis 1,2 bar.

Mit der direkten Temperaturregelung des Hauptreaktors 200 über den Kühlkreislauf 500 in Reihe mit dem ungekühlten Nachreaktor 210, hat sich überraschenderweise eine gegenüber dem Stand der Technik verringerter Energiebedarf und eine vereinfachte, stabilere Verfahrensführung gezeigt. Ohne auf eine spezielle Interpretation gebunden zu sein, wird dieser Erfolg darin gesehen, dass der Kühlkreislauf 500 nur gezielt für die sehr heftige Anfangsreaktion zur Ableitung der exothermen Reaktionswärme ausgelegt werden muss, wobei die noch bedeutsame Nachreaktion alleinig über die Zulauftemperatur mittels des stromaufwärts befindlichen Wärmetauscher 206 eingestellt werden muss. Der Stoffstrom wird durch die bereits stark abgeschwächte Reaktion im Nachreaktor 210 nur noch um ca. 5 bis 15 °C erwärmt und kann in diesem Niveau unproblematisch in den Trennkessel 300 entlassen werden.

Insgesamt sind in den Figuren in den Apparaten, wie Reaktoren, Trennkolonnen, Behältern etc., über die entsprechenden Symbole Einbauten, wie Packungen, Trennebenen, Trägerelemente etc. skizziert. Diese deuten jeweils vorteilhafte Ausführungsformen an, betreffend die Anzahl, Art und/oder relative Lage zur jeweils zu- oder abführenden Leitung. So ist bspw. mit der Darstellung ersten Trennkolonne 320 angedeutet, dass vorteilhafterweise der (Feed-)Stoffstrom über die Leitung 161 derart eingeleitet wird, dass zwischen dem Kopfumlauf und dem Sumpfumlauf jeweils mind. eine (theoretische) Trennebene vorhanden ist. Die Ermittlung der konkreten Art und/oder Anzahl von Trennebenen ist dem Fachmann bekannt und kann in geeigneter Weise variiert bzw. vorgesehen werden.

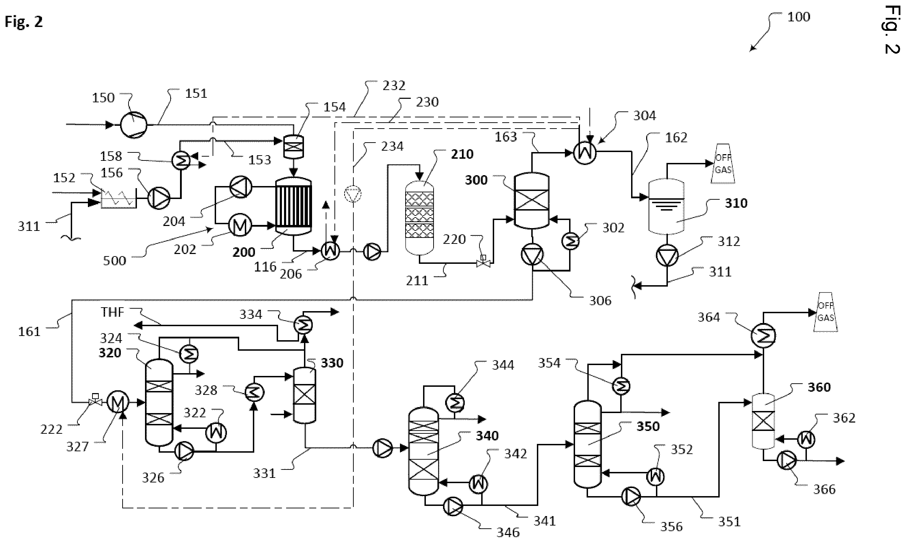

Figur 2 zeigt eine erste Ausführungsform einer direkten EK, wobei die Anlage 100 der aus Figur 1 entspricht. Die gestrichelten Leitungen 230, 232, 234 zeigen Medienleitungen, bei denen das erwärmte Medium vom Wärmetauscher 304 (Kondensator), angeordnet in der (Kopf-)Leitung 163 des Trennkessels 300, direkt zu stromabwärts befindlichen weiteren Wärmetauschern 158, 206 und 327 als Heizmedium weitergeleitet wird. Die drei Medienleitungen 230, 232, 234 und die damit verbundene direkte Medienweiterleitungen, können einzeln oder gemeinsam vorgesehen werden. Die Wärmetauscher 158, 206, 327 sind somit parallel zueinander geschaltet, wobei allen drei Wärmetauschern der Kondensator 304 als Wärmequelle dient. Das dem Kondensator 304 zufließende Medium, hier Wasser, verlässt stromabwärts zu den drei Wärmetauschern 158, 206, 327 die Anlage 100.

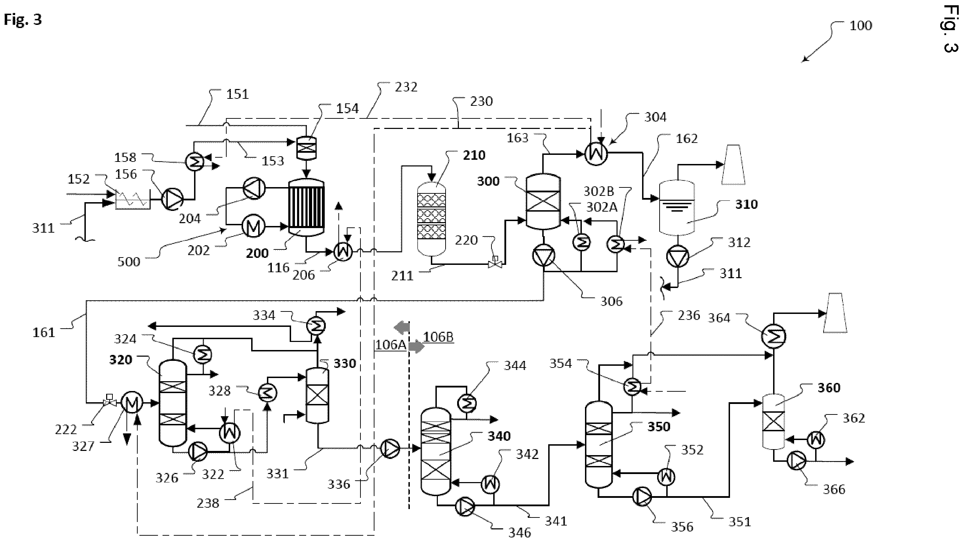

Figur 3 zeigt weitere Ausführungsformen für die direkte EK, als serielle mEK, wobei die Anlage 100 im Wesentlichen dem Figuren 1 und 2 entspricht. Analog zur Figur 2, ist der Wärmetauscher 304 als Wärmequelle mit den Wärmetauschern 158 im Zulauf zum Hauptreaktor 200 und dem (Vorlauf-)Wärmetauscher 327 in der (Feed-)Leitung 161 zur ersten Trennkolonne 320 verbunden. Die Wärmetauscher 158, 327 sind mit der Kondensator 304 in einer seriellen mEK und zueinander parallel geschaltet. Weiterhin ist der mit bspw. überhitztem Dampf beschickte (Sumpf-)Wärmetauscher 322 mit dem (Vorlauf-)Wärmetauscher 206 des Nachreaktors 210 für die Medienleitung 238 medienleitend verbunden. Das abströmende Kondensat oder die WasserDampf-Mischphase, verlässt den (Sumpf-)Wärmetauscher 322 bei einer Temperatur von über 170°C und dient für den (Vorlauf-)Wärmetauscher 206 durch direkte Energie-/Medienweiterleitung als Energiequelle, so dass für den Wärmetauscher 206 kein zusätzlicher Energieverbrauch erforderlich ist. Die Medienleitung 236 zeigt eine Medienverbindung zw. dem Wärmetauscher 354 der vierten Trennkolonne 350 und einem parallel geschalteten, zweiten (Sumpf-)Wärmetauscher 302B zu einem mit Dampf beschickten ersten Wärmetauscher 302A. Durch diese Redundanz mit zwei Sumpfwärmetauschern 302A,B im Sumpfkreislauf des Trennkessels 300, kann der dortige Energiebedarf um ca. 35 % gesenkt werden.

Im gezeigten Beispiel der Figur 3 sind die Wärmetauscher 304 (Kondensator) als Wärmequelle mit den als Wärmesenke arbeitenden Wärmetauschern 158, 206 und 327 über Medienleitungen in einer direkte EK, d.h. seriell verschaltet. Hierbei sind als Wärmesenken der Wärmetauscher 327 in der (Feed-)Leitung 161 zur ersten Trennkolonne 320, der Wärmetauscher 206 im Vorlauf des Nachreaktors 210 und der Wärmetauscher 158 in die (Feed-)Leitung 153 stromaufwärts zum Hauptreaktor 200 eingebunden. Diese Option einer direkten Wärmekopplung über Medienleitungen ist in dem unteren mit A gekennzeichneten Rahmen in der Figur 3 dargestellt. Diese selektive Direktverschaltung als direkte EK mittels Medienleitungen und Verteilung der Wärme des Kondensators 304, hat gegenüber der Einbindung der Wärmesenken in den Verteilerkreislauf 570 gem. bspw. Fig. 2, wie vorstehend beschrieben, eine Effizienzsteigerung von 30 bis 50 % zur Folge.

Vorliegend meint "Effizienzsteigerung", wenn nichts Abweichendes ausgeführt wird, einen geringeren Energieverbrauch. Der Bezug ergibt sich aus dem Zusammenhang und kann bezogen sein auf die Anlage, den jeweils beschriebenen Anlagenabschnitt oder den verbesserten Apparat bspw. durch Integration von zwei Wärmetauschern in einen einzigen.

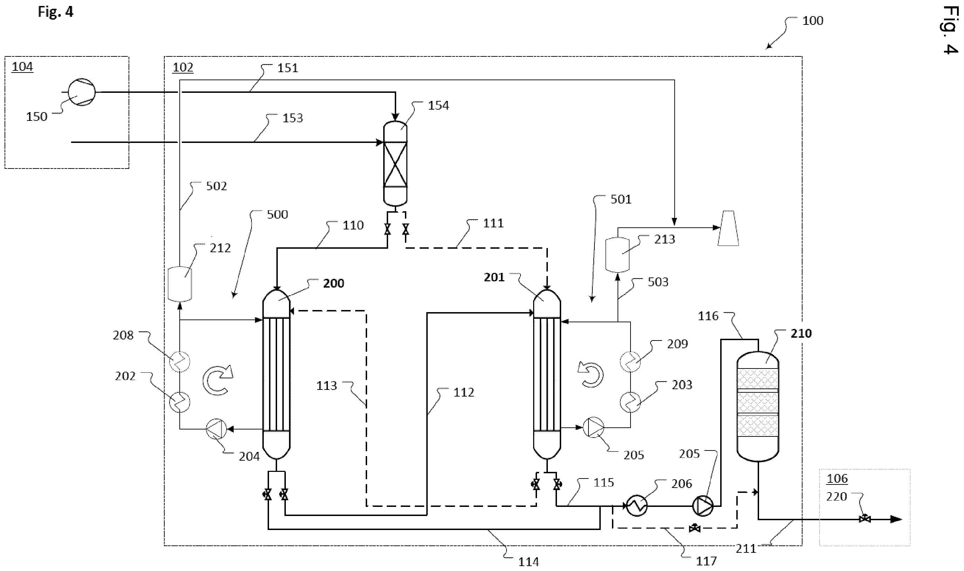

Figur 4 zeigt eine verbesserte Variante der Reaktoreinheit 102. Hierbei sind zwei Hauptreaktoren 200, 201 schaltbar zueinander in Reihe geschaltet. Die steuer- und/oder regelbaren Ventile/- einheiten sind zum Teil in der Figur 4 eingezeichnet, weitere können vom Fachmann bedarfsweise vorgesehen werden, um den sicheren Betrieb der beiden Hauptreaktoren 200, 201 sicherzustellen. Die beiden Hauptreaktoren 200, 201 sind jeweils in einen Kühlkreislauf 500, 501 eingebunden, mit dem jeweils der Festbettreaktor 200, 201 auf einer zulässigen, gekühlten Reaktionstemperatur gehalten wird. In der darstellten und mit vollausgezogenen Linien dargestellten Linien dargestellten Schaltung der Hauptreaktoren 200, 201 wird der erste Hauptreaktor 200 über die Leitung 110 kommend vom Mischbehälter 154 zuerst angeströmt und der erste Reaktionsanteil erfolgt in diesem Hauptreaktor 200. Stromabwärts über einen unteren Auslass und die Leitung 112 wird das Stoffgemisch in den Kopf des zweiten Hauptreaktors 201 geleitet und verlässt diesen über einen Bodenauslass und Leitung 115 in die gemeinsame Leitung 116 als Feed in den gemeinsamen Nachreaktor 210. In die gemeinsame Leitung 116 ist eine Wärmetauscher 206 eingebunden, worüber das für den Nachreaktor 210 erforderliche Temperaturniveau sichergestellt werden kann. Die Reihenfolge der Durchströmung der beiden Hauptreaktoren kann auch in umgekehrter Reihenfolge vom Hauptreaktor 201 zum Hauptreaktor 200 erfolgen. Hierzu wird in analoger Weise der Hauptreaktor 201 über die Leitung 111 zuerst angeströmt, wobei die Leitung 110 zum Kopf des Hauptreaktors 200 verschlossen ist. Das Stoffgemisch verlässt diesen Festbettreaktor über einen Bodenauslass und die Leitung (Zwischen-)Leitung 113, die mit dem Kopf des Hauptreaktors 200 verbunden ist, wobei die Leitung 115 verschlossen ist. Schließlich verlässt das Stoffgemisch den Hauptreaktor 200 über einen Bodenauslass und Leitung 114, die analog in die gemeinsame Leitung 116 mündet und damit zum Nachreaktor 210 führt. Aus beiden Kühlkreisläufen 500, 501 zweigt jeweils eine Leitung 502, 503 ab, die über jeweils einen Behälter 212, 213 geführt ist, die als Druckausgleichsbehälter dienen, und weiter zu einem Kamin und/oder einer vollständigen Oxidationseinheit. Die Leitungen und Ventile/-einheiten sind derart vorhanden und ausgelegt, dass jeder der Hauptreaktoren 200, 201 alleinige betrieben werden kann und der Stoffstrom um den jeweils anderen Hauptreaktor vollständig vorbeigeleitet werden kann. Die Fließrichtung der beiden Kühlkreisläufe 500, 501 ist durch einen Pfeil symbolisiert, wobei die beide Kühlkreisläufe 500, 501 vorteilhafterweise identisch oder im Wesentlichen gleich ausgebildet sind, da die beiden Hauptreaktoren 200, 201 abwechselnd bzgl. der Strömungsrichtung des Edukt- bzw. des Stoffstroms als erster bzw. zweiter Hauptreaktor betrieben werden.

Die Kühlkreisläufe 500, 501 sind allerdings derart ausgelegt und regelbar, dass je nach verfahrenstechnischen Bedarfen, wie insb. Produktleitung und/oder Produktqualität, jeweils eigenständige Kühlleistungen oder Kühlaufgaben erfüllt werden. Dies betrifft insb. den Volumenstrom pro Zeiteinheit an Kühlmedium und/oder das Temperaturniveau bzw. die zulässige Erwärmung des jeweiligen Kühlmediums. Weiterhin besteht eine direkte Möglichkeit der Wärmeintegration der Kühlkreisläufe 500, 501 mit anderen Analgenabschnitten oder Wärmetauschern, wobei die Wärmetauscher 202, 203 durch die Aufnahme der Reaktionswärme als Wärmequelle dienen, so dass insgesamt der Energieverbrauch der Anlag bzw. des Verfahrens zur Herstellung von PACM gesenkt werden kann.

In der Figur 4 ist weiterhin eine optionale Leitung 117 als gestrichelte Linie (Bypass 2) dargestellt, womit der Nachreaktor 210 umgangen werden kann, wenn beispielsweise dieser gewartet werden muss und/oder die Katalysatorfüllung erneuert werden muss. In diesem Fall wird mind. der in Strömungsrichtung des Stoffstroms zweite Hauptreaktor derart temperiert, dass die vollständige Reaktion bei der gewünschten Produktqualität, insb. dem gewünschten Anteil an den jeweiligen Isomeren sichergestellt ist. Der Leitungsabzweig der Leitung 117 kann in Strömungsrichtung vor oder nach Wärmetauscher 206 vorgesehen werden, wobei es vorteilhaft ist, diesen stromaufwärts zu Wärmetauscher 206 vorzusehen, um diese ggf. auch bypassen zu können und nötige Wartungsarbeiten bei laufender Anlage vornehmen zu können. Weiterhin besteht eine direkte Möglichkeit der Wärmeintegration der Kühlkreisläufe 500, 501 mit anderen Analgenabschnitten oder Wärmetauschern, wobei die Wärmetauscher 202, 203 durch die Aufnahme der Reaktionswärme als Wärmequelle dienen, so dass insgesamt der Energieverbrauch der Anlag bzw. des Verfahrens zur Herstellung von PACM gesenkt werden kann.

Die Umgehung des Hauptreaktors 200 kann im Betrieb des rechts im Bild dargestellten Hauptreaktors 201 über die Leitungen 111, 115 und 116 erfolgen. Die Umgehung des Hauptreaktors 201 kann im Betrieb des links im Bild dargestellten Hauptreaktors 200 über die Leitungen 110, 114 und 116 erfolgen, so dass jeweils die (Kreuz-)Leitungen 112, 113 zwischen den beiden Hauptreaktoren 200, 201 nicht durchflossen werden. In der Leitung 116 ist eine Pumpe 205 angeordnet.

In den Kühlumläufen 500, 501 sind weiterhin (optional) bei der dargestellten Anlagenvariante Wärmetauscher 208, 209 eingebunden. Dieser werden mit einem Heizmedium betrieben, insb. Dampf, und dienen im Anfahrschritt der Hauptreaktoren 200 der (Vor-)Temperierung auf Reaktionstemperatur der jeweiligen Hauptreaktoren 200, 201. Das Niveau dieser Vortemperierung liegt bei ca. 80 bis 100 °C, idealerweise 85 °C bis 95 °C. In dem gezeigten Anlagen- und Verfahrensbeispiel, wie bereits zur Figur 1 ausgeführt, ist es bei dem Ziel, ein möglichst niedriges trans/trans-Isomerenverhältnis von ca. 17 bis 23 Gew.% zur realisieren vorteilhaft, wenn die mit Katalysator neu befüllten Hauptreaktor 200, 201 auf eine Temperatur von ca. 90 °C mittels des jeweiligen Wärmetauschers 208, 209 vorgeheizt werden, bzw. der jeweilige neu befüllte Hauptreaktor 200, 201 entsprechend vorbeheizt wird.

Durch die Vorheizung auf typischerweise 85 bis 95 °C wird ermöglicht, dass die Reaktion bei dem vorliegenden Katalysator unmittelbar starten kann, ohne oder im Wesentlich ohne Recyclingströme des Stoffgemisches der Prozesses bis zum Erreichen der gewünschten Reaktionstemperatur.

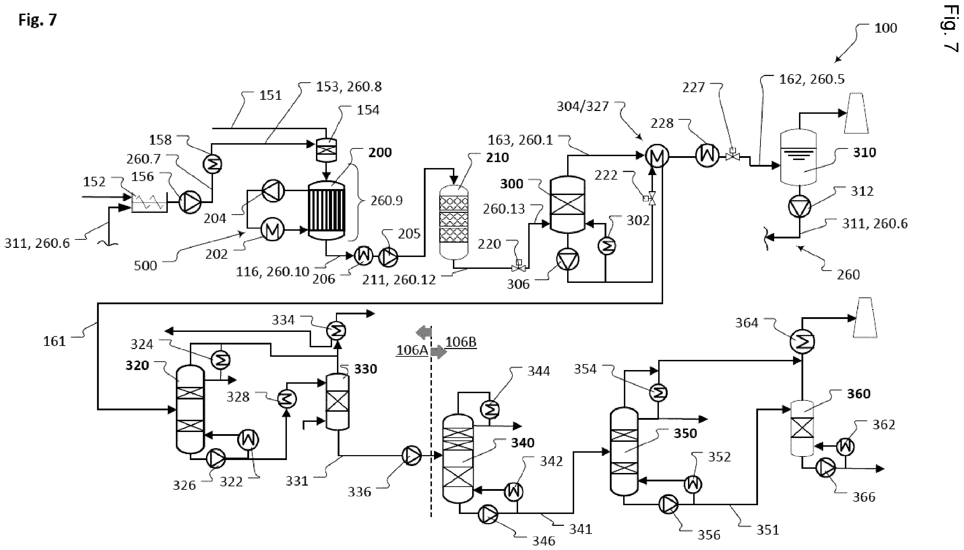

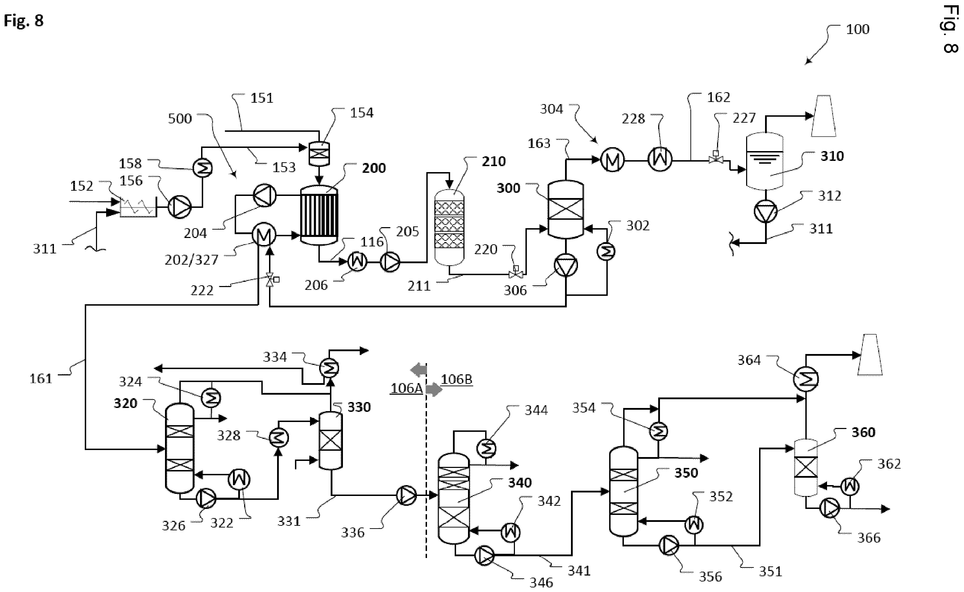

Die Variante der schaltbaren Hauptreaktoren 200, 201, wie sie in der Figur 8 dargestellt ist, unterscheidet sich zu der gem. Figur 7 darin, dass der Kühlkreislauf 500 des ersten Hauptreaktors 200 mit dem Kühlkreislauf des zweiten Hauptreaktors 201 ebenfalls in Reihe geschaltet ist. Hierbei ist nur ein (kühlender) Wärmetauscher 202 und nur eine Pumpe 204 für die gemeinsame Kühlung und den Medienumlauf vorgesehen, so dass der gemeinsame (zentrale) Leitungsast 508 in beiden Kühlkreisläufen 500, 501 in der Regel ständig und nur in eine Richtung durchströmt wird, unabhängig von der Verschaltung der beiden Hauptreaktoren 200, 201. Hierbei muss der "zentrale" Leitungsast natürlich nicht faktisch zwischen den beiden Hauptreaktoren 200, 201 angeordnet sein. Gezeigt ist die Variante als durchgezogenen Linien, bei der der erste Hauptreaktor 200 (links) zuerst mit dem Eduktgemisch über die Leitung 110 beschickt wird und auch die (Kühlmedien-)Einleitung nach dem Wärmetauscher 202 und der Pumpe 204 zuerst über diesen Hauptreaktor 200 erfolgt. Die in diese Schaltung nicht medienführenden Leitungen oder das Stoffgemisch führenden Leitungen sind gestrichelt dargestellt. Auch bei dieser Varianten besteht die Möglichkeit, das Stoffgemisch und/oder das (Kühl-)Medium vollständig an dem jeweils anderen Hauptreaktor vorbeizuleiten. Somit kann bspw. im Befüllvorgang eines der beiden Hauptreaktoren 200, 201, der jeweils andere unter maximaler Leistung weiter betrieben werden. Der Bypass des Stoffstroms bzw. des jeweiligen Hauptreaktors 200, 201 ergibt sich analog zu Figur 7.

In der gezeigten Beispielschaltung wird der zentrale Leitungsast 508 und der Wärmetauscher 202 durchflossen und über die Leitung 504 im Gleichstrom in den Medienraum ersten Hauptreaktors 200 geleitet, wobei zum gemeinsamen Leitungsknoten führende Leitung 505, kommend vom zweiten Hauptreaktor 201, gesperrt ist. Über die Leitung 506 verlässt das Medium an einem tiefen Auslass den ersten Hauptreaktor 202 und wird zu einem hohen Einlass in den Medienraum des zweiten Reaktors 201 geleitet (Kreuzleitung). Das Medium durchfließt den Medienraum des zweiten Hauptreaktors 201 ebenfalls im Gleichstrom und verlässt diesen an einem tiefliegenden Auslass über die Leitung 509, von der eine Abzweigung wieder in den zentralen Leitungsast 508 mündet, so dass der Kreislauf erneut durchflossen werden kann. Analog wird über die Leitung 505 der rechts dargestellte Hauptreaktor 201 zuerst durchströmt, wenn die Leitung 504 gesperrt ist. Das Medium verlässt dann den Medienraum des zweiten Hauptreaktors 201 über die Leitung 509 und wird an einem hochgelegenen Einlass in den Medienraum des anderen Hauptreaktors 200 geleitet. Der Auslass des Medienraums an einem tiefgelegenen Punkt führt in die Leitung 506 und von dort über eine Abzweigung in den zentralen Leitungsast 508.

Der besondere Vorteil des schaltbaren Hauptreaktors 200, 201 besteht in der Möglichkeit, den zuerst angeströmten Hauptreaktor bei einer höheren Temperatur zu betreiben, weil der Katalysator schon teilweise erschöpft und inaktiviert ist, im Wesentlichen ohne hierbei das trans/trans-Isomerenverhältnis negativ zu beeinflussen (d.h. zu erhöhen). Parallel hierzu fällt stärker erwärmtes Kühlmedium im jeweiligen (Umlauf-)Wärmetauscher 202, 203 des zuerst durchflossenen Hauptreaktors 200, 201 an. Dieses heißere Wärmetauschmedium kann aufgrund der höheren Temperatur besser in der Anlage zur integrierten medienbasierten EK und/oder zum üblichen Wärmetausch mit einem Stoff- Eduktstrom eingesetzt werden.

In der Figur 5 ist analoge zur Figur 4 die optionale Leitung 117 als gestrichelte Linie (Bypass 2) dargestellt, womit der Nachreaktor 210 umgangen werden kann, wobei die Leitung 117 stromabwärts zum (Vorlauf-)Wärmetauscher 206 des Nachreaktors 210 abzweigt.

In der Figur 5 ist weiterhin eine Anlagenvariante (gestrichelt) dargestellt, indem zur Erlangung einer höheren Regelungssicherheit bzw. eines höheren Freiheitsgrades des Temperaturmanagements in der jeweiligen (Kreuz-)Leitung 504, 505 der Kühlkreisläufe 500, 501 bedarfsweise schaltbare und regelbare Wärmetauscher 203 (Vorlaufkühler) angeordnet sind. In dem gezeigten Ausführungsbeispiel sind die beiden Wärmetauscher 202 in Reihe geschaltet, da durch die jeweils inaktive (Kreuz-)Leitung, indem gezeigten Beispiel die Leitung 505, am Einbauort für den Wärmetauscher 202 keine Erwärmung erfolgt. Die Kühlmedienleitung bzw. der Kühlmedienkreislauf der Wärmetauscher 202 kann bei einer vorteilhaften Variante in einem Seitenstrom bzw. über einen Neben- oder Hilfskreislauf über die Pumpe 204 betrieben werden.

Der Vorteil dieses Seitenstroms oder Neben-/Hilfskreislaufes eines Kühlmittels über die Wärmetauscher 203 hat den großen Vorteil, dass diese zusätzliche Kühlleistung nur bedarfsweise abgerufen werden braucht und mit geringem Aufwand eine weitergehend, bedarfsmäßige Regelung der Temperatur im jeweils zweiten beiden seriell geschalteten Hauptreaktoren erfolgen kann.

In der Figur 6 ist eine dritte Ausführungsform der Anlage mit einer weiteren direkten EK, als eine verbesserte Variante der bspw. in Figur 1 gezeigten Anlagenvariante. In der gezeigten Ausführungsform, sind insg. drei Wärmetauscher in integrierter stoffbasierter EK gezeigt. Um den Bezug zur Figur 1 herzustellen, ist in den Figuren insgesamt die Schreibweise "/" bei Wärmetauschern in integrierter medienbasierter EK oder integrierter stoffbasierter EK genutzt, wie bspw. Kondensator 304/158, womit ausgesagt wird, dass mind. ein Teil des Wärmeübertrages von vormals zwei Wärmetauschern nunmehr in einem einzelnen Wärmetauscher erfolgt. Die Reihenfolge der Bezugszeichen ist hierbei ohne weitere Bedeutung. In dem Beispiel der Figur 6 wird die vollständige Temperaturerhöhung des Stoffstroms der (Feed-)Leitung 153 vollzogen, wohingegen die Abkühlung des Stoffstroms der Leitung 163 unvollständig sein kann, so dass eine Wärmetauscher 228 als mind. optionaler Nachkondensator vorgesehen ist. Dieser (Bedarfs-)Wärmetauscher 228 stromabwärts zum Kondensator 304 in der Leitung 163, 162 kann baulich kleiner sein, weil die Kühlaufgabe vergleichsweise gering ist. Im Idealfall erfolgt der vollständige Wärmeaustausch für beide Stoffströme in einem einzigen Wärmetauscher bei der integrierten stoffbasierten EK. Vorliegend soll die Leitung 163 regelmäßig die gesamte Leitung vom Trennkessel 300 bis zum Sammelbehälter 310 meinen und die Leitung 162 den Leitungsabschnitt zw. dem Kondensator 304 zum Sammelbehälter 310.

Als weitere integrierte stoffbasierte EK ist der Stoffstrom der (Kopf-)Leitung 347 der dritten Trennkolonne 340 mit dem Stoffstrom der (Feed-)Leitung 161 zur ersten Trennkolonne 320 in dem Wärmetauscher 327/344 gekoppelt, wobei analog zur o.g. Variante ein sehr viel kleinerer (Kopf-)Kondensator 344.1 am Kopf der dritten Kolonne 340 vorgesehen ist, um die erforderliche Kondensation des Dampfes im Kopf der Trennkolonne 340 sicherzustellen. Die dritte integrierte stoffbasierte EK erfolgt zwischen dem Stoffstrom der (Kopf-)Leitung 357 der vierten Trennkolonne 350 mit dem Stoffstrom der (Feed-)Leitung 116 zum Nachreaktor 210. Diese dritte integrierte stoffbasierte EK erfolgt im Wärmetauscher 206/354. Die Veränderung der Temperaturniveaus in den jeweiligen Stoffströmen erfolgt analog, wie vorstehend beschrieben. In analoger Weise zur zweiten integrierten stoffbasierten EK, wobei im Rücklauf ein sehr viel kleinerer (Kopf-)Kondensator 354.1 am Kopf der vierten Kolonne 340 vorgesehen ist, um die erforderliche Kondensation des Dampfes im Kopf der Trennkolonne 350 dauerhaft oder bedarfsweise sicherzustellen.

In der Figur 7 ist eine weitere direkte EK der Anlage 100 dargestellt, wobei der Stoffstrom der (Kopf-)Leitung 163 des Trennkessels 300 im Kondensator 304 als integrierte stoffbasierte EK mit dem Stoffstrom aus dem (Sumpf-)Auslass über die Leitung 161 des Trennkessels 300 wärmetauschend gekoppelt ist. Im Unterschied zur Figur 1, ist bereits stromaufwärts zum Kondensator 304 in der Leitung 161 eine Druckregeleinheit 222 angeordnet, um durch Entspannung den erforderlichen Temperaturgradienten in dem zufließenden Stoffstrom für den Kondensator 304 einregeln zu können. Die Leitung 161 ist stromabwärts zum Kondensator 304 mit der ersten Trennkolonne 320 verbunden. Die (Kopf-)Leitung 163 ist stromabwärts vom Kondensator 304 mit dem Sammelbehälter 310 verbunden. Analog zur Figur 6, kann optional in der (Kopf-)Leitung 163 eine Druckregeleinheit 227 und/oder eine bedarfsweise schaltbarer Wärmetauscher 228 angeordnet sein, um die vollständige Kondensation der Dampfphase in der Leitung 162, 163 stromaufwärts zum Sammelbehälter 310 sicher zu stellen.

Bei dieser Ausführungsform kann für den wärmetauschenden Stoffstrom ein Kreislauf 260 ausgebildet werden, der mehrere Leitungsabschnitte 260.n umfasst und in den der Mischer 152, Hauptreaktor 200, der Nachreaktor 210, der Trennkessel 300 in den wärmetauschenden Stoff- oder Eduktstrom eingebunden sind. Weiterhin sind als einer der Leitungsabschnitte 260.n die (Kopf-)Leitung 163, die (Feed-)Leitung 162, die (Rück-)Leitung 311 sowie die (Feed-)Leitung 116 eingebunden. Als wärmetauschender Stoff- oder Eduktstrom dient das Lösungsmittel bzw. der Anteil an Lösungsmittel im jeweiligen Strom.

Diese Direktverschaltung als EK der Stoffströme und Verteilung der Wärme des Kondensators 304, hat gegenüber der Einbindung der Wärmesenken in den Verteilerkreislauf 570 gem. Fig. 2, wie vorstehend beschrieben, eine Effizienzsteigerung von 30 bis 50 % zur Folge.

In der Figur 8 ist im Unterschied zu Figur 7 und der dortigen Ausführungsform, eine weitere EK der Anlage 100 dargestellt, wobei der Stoffstrom der (Sumpf-)Leitung des Trennkessels 300 bzw. der (Feed-)Leitung 161 zur ersten Trennkolonne 320 wärmetauschend mit dem (Umlauf-)Wärmetauscher 202 im Kühlmittelumlauf 500 des Reaktors 200 gekoppelt ist. Im Unterschied zur Figur 1 und analog zu den Beispielen der Figur 6 und Figur 7, ist bereits stromaufwärts zum (Umlauf-)Wärmetauscher 202 in der Leitung 161 eine Druckregeleinheit 222 angeordnet, um durch Entspannung den erforderlichen Temperaturgradienten in dem zufließenden Stoffstrom für den Kondensator 304 einregeln zu können. Die Leitung 161 ist stromabwärts zum (Umlauf-)Wärmetauscher 202 mit der ersten Trennkolonne 320 verbunden.

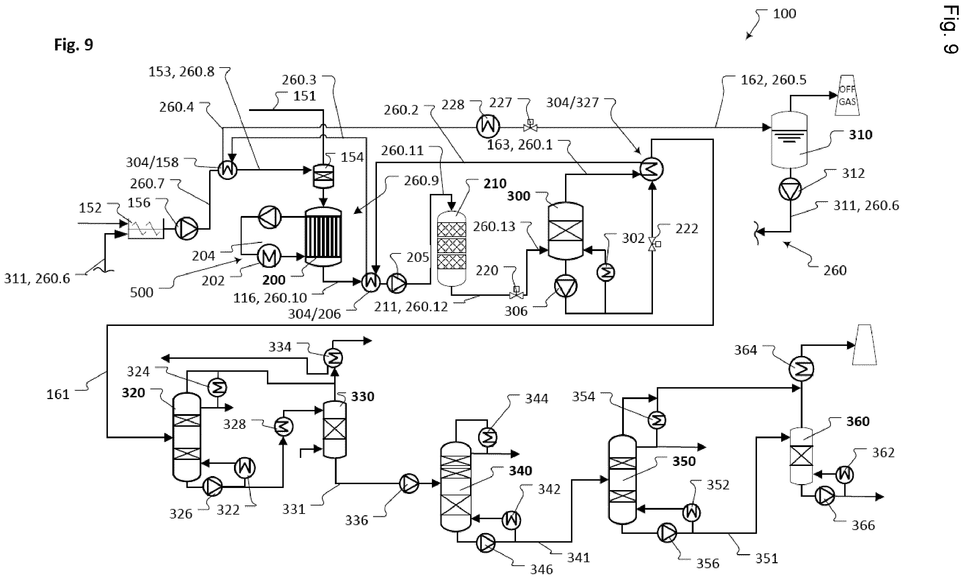

In der Figur 9 ist eine weitere direkte EK der Anlage 100 dargestellt, wobei analog zur Variante nach Figur 7, der Stoffstrom der (Kopf-)Leitung 163 des Trennkessels 300 im Kondensator 304 als integrierte stoffbasierte EK mit dem Stoffstrom aus dem (Sumpf-)Auslass über die Leitung 161 des Trennkessels 300 wärmetauschend gekoppelt ist. Stromaufwärts zum Kondensator 304 ist in der Leitung 161 eine Druckregeleinheit 222 angeordnet, um durch Entspannung den erforderlichen Temperaturgradienten in dem zufließenden Stoffstrom für den Kondensator 304 einregeln zu können. Die Leitung 161 ist stromabwärts zum Kondensator 304 mit der ersten Trennkolonne 320 verbunden. Die (Kopf-)Leitung 163 ist stromabwärts vom Kondensator 304 mit dem Sammelbehälter 310 verbunden. Optional kann in der (Kopf-)Leitung 163 eine Druckregeleinheit 227 und/oder eine bedarfsweise schaltbarer Wärmetauscher 228, um die vollständige Kondensation der Dampfphase in der Leitung 162, 163 stromaufwärts zum Sammelbehälter 310 sicher zu stellen. Im Unterschied zur Figur 7, ist die (Kopf-)Leitung 163 aber nicht stromabwärts über die (Feed-)Leitung 162 unmittelbar mit dem Sammelbehälter 310 verbunden. Der Wärmetauscher 304/327 ist seriell als integrierte stoffbasierte EK mit den als Wärmesenke fungierenden Wärmetauschern 206, 304 bzw. der (Feed-)Leitung 116 zum Nachreaktor 210 und stromabwärts ebenfalls seriell mit dem Wärmetauscher 158/304 als integrierte stoffbasierte EK verbunden, wobei der abführende Leitungsast der (Feed-)Leitung 162 zum Sammelbehälter 310 entspricht. Diese energetische Verschaltung zur Weiterleitung und Verteilung der Wärmeenergie, ist auch in der Variante als Wärmetauschkreislauf 260 dargestellt, wobei zum leichteren Verständnis die Leitungsabschnitte 260.n des Kreislaufs 260 als 260.1 bis 260.13 durchlaufend nummeriert sind. Die rückführende Leitung 311 entspricht dem Leitungsast 260.6 und leitet das wärmeleitende Lösungsmittel, hier bspw. THF, aus der ersten Trennstufe 106A zurück in die Konditionierungseinheit 104. Ggf. vorteilhafte Sammel- oder Lagertanks der Konditionierungseinheit 104 sind optional in den Wärmetauschkreislauf 260 einbindbar.

Bei dieser Ausführungsform kann für den wärmetauschenden Stoffstrom ein Kreislauf 260 ausgebildet werden, der mehrere Leitungsabschnitte 260.n umfasst und in den der Mischer 152, Hauptreaktor 200, der Nachreaktor 210, der Trennkessel 300 in den wärmetauschenden Stoff- oder Eduktstrom eingebunden sind. Weiterhin sind als einer der Leitungsabschnitte 260.n die (Kopf-)Leitung 163, die (Feed-)Leitung 162, die (Rück-)Leitung 311 sowie die (Feed-)Leitung 116 eingebunden. Als wärmetauschender Stoff- oder Eduktstrom dient das Lösungsmittel bzw. der Anteil an Lösungsmittel im jeweiligen Strom.

Als besonders überraschend und vorteilhaft hat sich Einbindung und integrierte stoffbasierte EK des (Vorlauf-)Wärmetauscher 327 herausgestellt stromaufwärts zur ersten Trennkolonne 320, weil diese nach der Druckregeleinheit 222 in der Leitung 161 auf einem sehr niedrigen Druckniveau von ca. 1,1 bar bis 2 bar betrieben werden kann und damit das Temperaturniveau des Stoffstroms, der dem Wärmetauscher 327 zuströmt sehr tief ist, bei ca. 90 °C. Damit können relative große Wärmemengen bei Energiekopplung aufgenommen und Kühlleistung von eingespart werden, weiterhin ist der Energiebedarf des Sumpfkreislaufs der ersten Trennkolonne 320 fast linear. Bei der integrierten stoffbasierten EK kann auf diesem Wege bezogen auf den Gesamtenergiebedarf der zweiten Trennkolonne 320 bspw.

| | |
|---|---|
| - des Wärmetauschers 327/344 | ca. 31 %, |
| - des Wärmetauschers 327/354 | ca. 62 %, |
| - des Kondensators 327/304 | ca. 21 % und/oder |
| - des (Umlauf-)Wärmetauschers 327/202 | ca. 12 % |

eingespart werden.

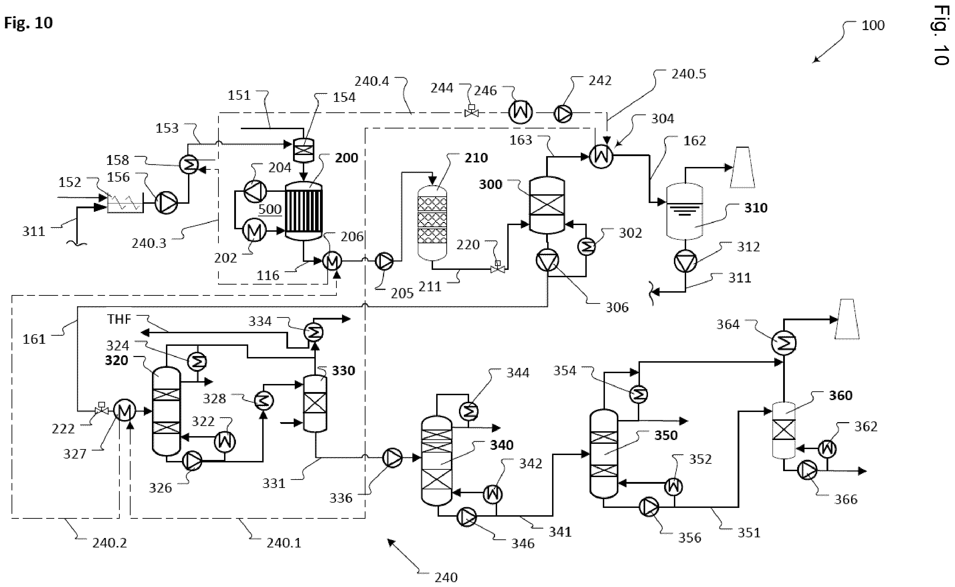

Die Figur 10 zeigt eine Ausführungsform bzw. Varianten, die analog zu den Varianten der Figuren 2, 3 ausgebildet sind. Hierbei ist ein Wärmekreislauf 240 als serielle Verschaltung Wärmetauschern zur integrierten medienbasierten EK ausgebildet. Hierbei dient der Kondensator 304 als zentrale Wärmequelle und über n Leitungsabschnitte 240.n, der (Vorlauf-)Wärmetauscher 327 in der (Feed-)Leitung 311, der (Feed-)Wärmetauscher 206 in der (Feed-)Leitung 116 und der Wärmetauscher 158 in der (Feed-)Leitung 153 eingebunden sind. Als Wärmetauschmedium dient bspw. Wasser, das in den Leitungsabschnitten 240.n im Kreis geführt wird. Die Leitungsabschnitte eines Wärmetauschkreislauf 240, aber auch der vorherig genannten Wärmetauschkreisläufe 250, 260 zeichnen sich dadurch aus, dass im n-ten Leitungsabschnitt, gegenüber dem Leitungsabschnitt n-1 (stromaufwärts) und/oder Leitungsabschnitt n+1 (stromabwärts) ein unterschiedliche Temperaturniveau und/oder ein unterschiedliches Druckniveau vorliegt. In den gezeigten Beispielen und Varianten sind hierbei die dargestellten und/oder ausgewiesenen Leitungsabschnitte exemplarisch dargestellt, so dass weitere aufgrund der jeweiligen Bedarfe vorgesehen sein können, insb. wenn weitere Wärmetauscher als Wärmesenke und/oder weitere Wärmetauscher als Wärmequelle in den Wärmetauschkreislauf 240, 250, 260 in analoger Weise eingebunden werden.

In den Leitungsabschnitten 240.4, 240.5 ist in der gezeigten Variante der Anlage 100 eine Druckregeleinheit 244 und ein als Wärmesenke arbeitende Wärmetauscher 246 stromaufwärts zum Kondensator 304 angeordnet, um die erforderliche Kühl-/Kondensationsleistung des Kondensators 304 zu gewährleisten.

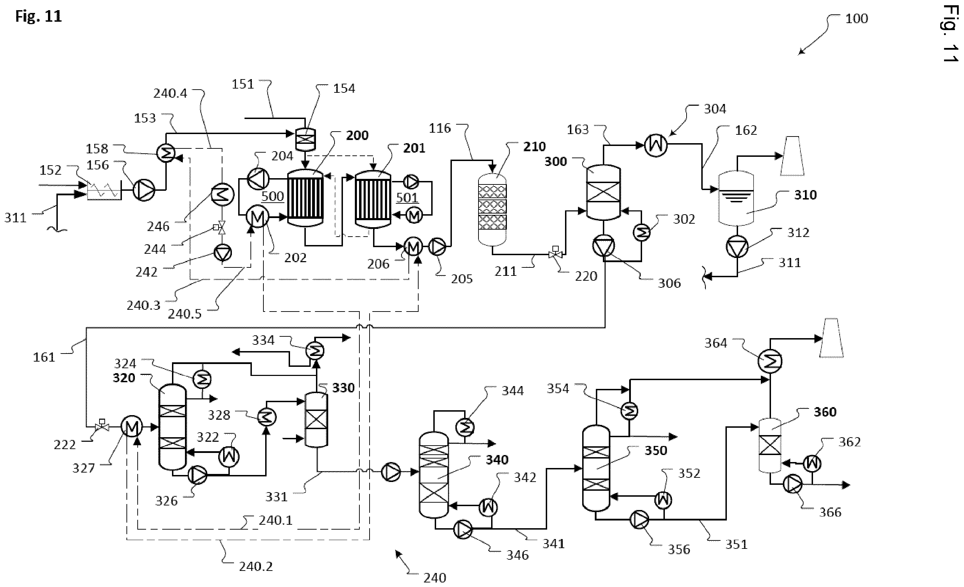

Das Ausführungsbeispiel der Figur 11 entspricht schließlich im Grundaufbau dem der Figur 10, wobei im Unterschied hierzu, nicht der Kondensator 304 stromabwärts des Trennkessels 310, sondern der (Kreislauf-)Wärmetauscher 202 des Reaktors 200 als zentrale Wärmequelle des Wärmetauschkreislaufs 240 dient. In analoger Weise ist im Übergang vom Leitungsabschnitten 240.4 zum letzten Leitungsabschnitt 240.5 vor dem (Umlauf-)Wärmetauscher 202 eine Druckregeleinheit 244 und ein als Wärmesenke (kühlender) arbeitende Wärmetauscher 246 angeordnet, um die erforderliche Kühlleistung des (Umlauf-)Wärmetauschers 202 zu gewährleisten und somit den Reaktor 200 optimal regeln zu können.

Ein bedeutsamer Vorteil des (Verteiler-)Kreislaufes 240 kann darin gesehen werden, dass ein zentraler Dampfkreislauf geschaffen wurde und somit Dampf zentral in bspw. einem Kondensator 304 erzeugt und auf alle als Verbraucher (Wärmesenke) arbeitenden Wärmetauscher verteilt werden kann, anstelle der direkten Verschaltung von Wärmequellen und Wärmesenken. Weiterhin kann der Verteilerkreislauf als zentraler Dampfkreislauf im Bedarfsfall (z.B. beim Anfahren der Anlage) mindestens zeitweise mittels einer alternativen Wärme- oder Dampfquelle gespeist werden.

Der (Vorlauf-)Wärmetauscher 206 ist vorliegend vorrangig beschrieben und teilweise in einer EK gezeigt, bei denen dieser als Wärmetauscher 206 vorrangig als Wärmesenke arbeitet, also der darin geführte Stoffstrom erwärmt wird. Durch die an den Hauptreaktor 200, 201 angepasste, abhängige Betriebsweise des Nachreaktors 210 kann mind. zeitweise, insb. dauerhaft, eine Kühlung des Stoffstroms in der (Feed-)Leitung 116 stromaufwärts zum Nachreaktor 210 erforderlich sein, weil im (adiabatischen) Nachreaktor ca. 10 bis 20% des Umsatzes erfolgen, so dass eine Erwärmung des Stoffstroms gemessen im Auslass bis auf ca. 140 °C erfolgt. Somit kann unabhängig von den hierhin beschriebenen Ausführungsformen und Varianten der Anlagen und des Verfahrens eine angepasste EK zur Kühlung (Wärmetauscher 206 arbeitet als Wärmequelle) vorgesehen werden. Alternativ oder zusätzlich kann eine ergänzende Kühlung über eine geänderte oder weitere EK vorgesehen werden.

Insgesamt sind eine Vielzahl von üblichen, dem Fachmann bekannte und für eine vorteilhafte Prozessführung nötige oder sinnvolle Steuer- und Regelungselemente nicht dargestellt, wie Sensoren (Durchfluss, Temperatur, Druck etc.), Anzeigen, Stell- und Regelglieder (insb. Ventile, weitere Pumpen, Verdichter), Sammelbehälter etc. und sind bedarfsweise zu ergänzen. Insb. sind auch bei der Nennung von "einer" Pumpe oder "einem" Verdichter übliche Redundanzen aus mind. zwei parallelen Aggregaten gemeint, insb. von zwei parallelen Pumpen oder zwei parallelen Verdichtern. In analoger Weise ist "ein Wärmetauscher" nicht limitierend zu verstehen und meint mit der jeweiligen, örtlichen Wärmetauschaufgabe auch Anordnungen von in Reihe geschalteten Wärmetauschern oder parallel geschaltete, auch redundanten, Wärmetauschern, wobei hiermit keine Verschaltungen mit mind. einem weiteren Wärmetauscher und örtlich unterschiedlicher Wärmetauschaufgabe zu verstehen ist.

Grundsätzlich sind alle Wärmetauscher und Kondensatoren derart ausgebildet, dass ein indirekter Wärmeübertrag erfolgt und keine stoffliche Vermischung von Edukten, Produkt, Nebenprodukten und/oder Lösungsmittel mit den (Heiz-/Kühl-)Medium, wie Gas, Dampf, Wasser, Öl, Sole (Brine) etc. erfolgt, es sei denn es ist etwas hiervon Abweichendes ausgeführt.

Auch wenn vorliegend Bauteile, wie Ventile, Druckregeleinheit, Absperraggregate etc. zur vereinfachten Beschreibung einzeln oder gesondert dargestellt sind und zum Teil nicht einzeln genannt wurde, ist dies nicht limitierend zu lesen, vielmehr kann der Fachmann bedarfsweise zwei oder mehrere dieser Bauteile in einer Ventil- oder Stelleinheit zusammenfassen oder Mehrwegventile stattdessen vorsehen.

Aufgrund der Betrachtung von Medienströmen, Edukt- und Stoffströmen zur EK, sind die Begriffe "stromaufwärts" oder "stromabwärts" nur aus dem jeweiligen Textzusammenhang interpretierbar.

Die Begriffe "Sumpfablauf", "Sumpfauslauf", "Sumpfableitung" oder "Sumpfausleitung" werden zum Teil synonym verwendet, ebenso werden in analoger Weise die Begriffe "Kopfablauf, - auslauf, - ableitung oder -ausleitung" zum Teil synonym verwendet.

Weiterhin ist der Begriff "Wärmetauscher, der als Kondensator betrieben wird" breit auszulegen, und meint auch eine unvollständige Kondensation oder Abkühlung des zugeleiteten Stoffstroms, so dass ein "Wärmetauscher, der als Kondensator betrieben wird", auch teilweise synonym als "Kondensator" benannt wird.

Der Begriff "Sumpfpumpe" meint eine in einen Sumpfumlauf eines Apparates (Trennkolonne, Behälter, etc.) eingebundene Pumpe und/oder eine sich stromabwärts zum Sumpfauslass eines Apparates befindliche Pumpe, die zur Förderung flüssigen Stoffstroms vorgesehen ist.

Über die hierin gezeigten Beispiele hinaus, hat es sich insgesamt als überraschend vorteilhaft und effektiv herausgestellt, stromabwärts der Druckentspannungseinheit einen Vorlaufwärmetauscher in der (Feed-)Leitung zur ersten Trennkolonne der ersten Trennstufe vorzusehen, vorteilhafterweise diesen in einer EK vorzusehen, insb. in einer integrierten EK vorzusehen.

Für alle Ausführungsformen und Varianten der Anlage und des Verfahrens kann generell vorgesehen werden, dass die zweite Trennkolonne 340 und die dritte Trennkolonne 350 als eine einzige Kolonne ausgeführt werden, insb. als eine Trennwandkolonne (nicht dargestellt). Hierbei können vorteilhafterweise die Trennaufgaben der zweite Trennkolonne 340 und der dritten Trennkolonne 350 mind. teilweise, idealerweise vollständig mittels der Trennwandkolonne (nicht dargestellt) ausgeführt werden, wie sie bspw. aus den Druckschriften EP 012 62 88 B1 oder EP 012 23 67 A2 bekannt sind.

Insgesamt kann ein großer, energetischer Vorteil mit der erfindungsgemäßen Anlage und dem Verfahren erreicht werden, der darin besteht, dass eine starke Verringerung der extern zugeführten Energieströme ermöglicht wurde, insb. die Einsparung von großen Mengen an (externem) Heizdampf.

## Patentansprüche

1. Anlage (100) zur katalytischen Hydrierung von Methylendianilin (MDA; Edukt1) mit einem Wasserstoffgeber (Edukt2), insb. einem gasförmigen Wasserstoffgeber, vorzugsweises Wasserstoff (H2),
umfassend eine Konditionierungseinheit (104) für die Edukte, eine Reaktoreinheit (102) und eine Trenneinheit (106), wobei
- die Konditionierungseinheit (104) (Zu-)Leitungen für Edukte1, Edukt2 und mind. ein Lösungsmittel, mind. einen Wärmetauscher (158) in mind. einer (Zu-)Leitung, mind. einen Mischer (152) zur Mischung der Edukte und/oder mind. eines Edukts mit mind. einem Lösungsmittel umfasst;
- die Reaktoreinheit (102) mind. einen Festbettreaktor als Hauptreaktor (200, 201) mit einer immobilen Katalysatorpackung umfasst, wobei der mind. eine (erste) Hauptreaktor (200, 201)
- einen ersten Strömungsweg für das Stoffgemisch
über die immobile Katalysatorpackung und
- einen weiteren Strömungswegumfasst, und
wobei in den weiteren Strömungsweg ein Wärmetauscher (202) eingebunden ist, zur Beeinflussung des Temperaturniveaus in dem ersten Strömungsweg;
- die Trenneinheit (106) umfassend mind.
- eine erste Trennstufe (106A), umfassend mind. einen Apparat (300, 320) zur Abtrennung des Lösungsmittels, wobei der mind. eine Apparat (300, 320) über mind. eine (Kopf-)Leitung (163) mit mind. einem Kondensator (304) stromabwärts verbunden ist, und
- eine zweite Trennstufe (106B), umfassend mind. einen Apparat (340, 350, 360) zur Abtrennung vom mind. einem Edukt und/oder mind. einem Nebenprodukt vom Produkt, wobei der mind. eine Apparat (340, 350, 360) über mind. eine (Kopf-)Leitung Q mit mind.
einem Kondensator (344, 354) stromabwärts verbunden ist,
**dadurch gekennzeichnet, dass**
der weitere Strömungsweg ein geschlossener Medienumlauf (500) für ein Wärmeträgermedium ist, der mind. auf einer Teilstrecke außerhalb der Katalysatorpackung des mind. einen Hauptreaktors (200) zum indirekten Wärmeübergang verläuft, wobei in den Medienumlauf (500) ein Wärmetauscher (202) eingebunden ist.

2. Anlage nach einem der vorherigen Ansprüche, **dadurch gekennzeichnet, dass** die **Reaktoreinheit** (102) zum mind. einen Hauptreaktor (200) mind. einen stromabwärts befindlichen seriell verbundenen Nachreaktor (210) umfasst, wobei der mind. eine Hauptreaktor (200) und der mind. eine Nachreaktor (210) über eine Leitung (116) eingebunden ist, insb. eine Leitung (116), in die ein Wärmetauscher (206) eingebunden ist.

3. Anlage nach Anspruch 1 oder 2, **dadurch gekennzeichnet, dass** mind. eine Energiekopplung (EK) vorgesehen ist, in die mind. einer der folgenden Wärmetauscher eingebunden ist:
- der mind. eine Kondensator (304) der ersten Trennstufe (106A),
- der mind. eine Kondensator (344, 354) der zweiten Trennstufe (106B),
- der mind. eine Wärmetauscher (202) des Medienumlaufs (500), und wobei die EK folgendes meint:
i) eine integrierte stoffbasierte Energiekopplung (integrierte stoffbasierte EK) von mind. zwei Stoffströmen in einem Wärmetauscher im indirekten Wärmeaustausch;
ii) einen integrierte medienbasierte Energiekopplung (integrierte medienbasierte EK) von mind. zwei Wärmetauschmedien in einem Wärmetauscher im indirekten Wärmeaustausch;
iii) eine serielle Verschaltung von zwei Wärmetauschern in medienbasierter Energiekopplung (serielle mEK), wobei
- in serieller Verschaltung vom ersten Wärmetauscher in integrierter medienbasierter EK oder allgemeiner EK mit einem ersten Stoffstrom das (Wärmetausch-)Medium zum zweiten stromabwärts befindlichen Wärmetauscher zur weiteren integrierten medienbasierten EK oder allgemeinen EK mit einem weiteren Stoffstrom weitergeleitet wird.

4. Anlage nach Anspruch 3, **dadurch gekennzeichnet, dass** die Anlage (100) folgendes umfasst:
iv) mind. eine serielle Verschaltung von zwei Wärmetauschern zur integrierten stoffbasierten EK (kurz "serielle integrierte stoffbasierte EK"), wobei in serieller Verschaltung ein Stoffstrom vom ersten Wärmetauscher in integrierter stoffbasierter EK zum zweiten stromabwärts befindlichen Wärmetauscher zur weiteren integrierten stoffbasierten EK weitergeleitet wird.

5. Anlage nach Anspruch 3 oder 4, **dadurch gekennzeichnet, dass** mind. eine serielle medienbasierte Energiekopplung (mEK) nach iii) oder eine serielle integrierte medienbasierte EK nach iv) als Kreislauf, insb. als geschlossener Kreislauf ausgebildet ist.

6. Anlage nach einem der vorherigen Ansprüche 3 bis 5, **dadurch gekennzeichnet, dass** mind.
einer der nachfolgenden Leitungen im Kondensator (304) in der (Kopf-)Leitung (163) des Trennkessels (300) als Wärmequelle zur integrierten stoffbasierten EK wie folgt eingebunden sind:
i) die (Feed-)Leitung (153) zum Hauptreaktor (200)
ii) die (Feed-)Leitung (116) zum Nachreaktor (210),
iii) die (Feed-)Leitung (161) zur ersten Trennkolonne (320), wobei in der (Feed-)Leitung (161) stromaufwärts zum Kondensator (304) eine Druckregelungseinheit (222) angeordnet ist,
iv) stromabwärts zu einer Einbindung einer Leitung nach i), ii) oder iii) im Kondensator (304) und stromaufwärts zu einem Sammelbehälter (310) mind. ein weiterer Wärmetauscher zur integrierten stoffbasierten EK eingebunden ist.

7. Anlage nach einem der vorherigen Ansprüche, **dadurch gekennzeichnet, dass** die Anlage (100) eine serielle mEK als Kreislauf (260) mit einer Mehrzahl an Leitungsabschnitten (260.n) umfasst, wobei mind. die folgenden Apparate und/oder Leitungen eingebunden sind:
der Sammelbehälter (310) stromabwärts zum Trennkessel (300), die (Kopf-)Leitung (163) als ein Leitungsabschnitt (260.1), insb. ein erster Leitungsabschnitt (260.1), die (Feed-)Leitung (162) zum Trennkessel (310) als ein weiterer Leitungsabschnitt (260.5), insb. ein letzter Leitungsabschnitt (260.5).

8. Anlage nach Anspruch 7, **dadurch gekennzeichnet, dass** der Kondensator (304) nach Anspruch 6 Alternative iii) in integrierter stoffbasierter EK steht und in den Kreislauf (260) eingebunden ist.

9. Anlage nach einem der vorherigen Ansprüche 3 bis 8, **dadurch gekennzeichnet, dass** als Wärmequelle der Medienumlauf (500) des Hauptreaktors (200) im Wärmetauscher (202) mit der (Feed-)Leitung (161) zur ersten Trennkolonne (320) zur EK eingebunden sind, wobei in der (Sumpf-)Leitung (161) stromaufwärts zum Wärmetauscher (202) eine Druckregelungseinheit (222) angeordnet ist.

10. Anlage nach einem der vorherigen Ansprüche 3 bis 9, **dadurch gekennzeichnet, dass** mind. eine der nachfolgenden Leitungen in einem Kondensator (344, 354) einer Trennkolonne (340, 350) der zweiten Trennstufe (106B) in der jeweiligen (Kopf-)Leitung (234, 236) als Wärmequelle zur integrierten stoffbasierten EK wie folgt eingebunden sind:
i) die (Feed-)Leitung (153) zum Hauptreaktor (200),
ii) die (Feed-)Leitung (116) zum Nachreaktor (210),
iii) die (Feed-)Leitung (161) zur ersten Trennkolonne (320) der ersten Trennstufe (106B), wobei in der (Feed-)Leitung (161) stromaufwärts zum Kondensator (344, 354) der jeweiligen Trennkolonne (340, 350) eine Druckregelungseinheit (222) angeordnet ist.

11. Anlage nach einem der vorherigen Ansprüche, **dadurch gekennzeichnet, dass** mind. eine direkte Medienleitungen (230, 232, 234) zwischen Wärmetauschern zur direkten Energiekopplung (direkte EK) vorgesehen ist:
i) Kondensator (304) der Trenneinheit (106) als Wärmequelle für mind. einen Wärmetauscher der Reaktoreinheit (102), der Konditionierungseinheit (104) und/oder der Trenneinheit (106),
ii) Wärmetauscher (202) des Medienumlaufs (500) zum Hauptreaktor (200) der Reaktoreinheit (102) als Wärmequelle für mind. einen Wärmetauscher der Reaktoreinheit (102), der Konditionierungseinheit (104) und/oder der Trenneinheit (106) und/oder
iii) Wärmetauscher der der Reaktoreinheit (102), der Konditionierungseinheit (104) und/oder Trenneinheit (106), untereinander.

12. Anlage nach Anspruch 11, **dadurch gekennzeichnet, dass** die Medienleitung zur direkten EK:
i) vom Kondensator (304) des Trennkessels (300) der Trenneinheit (106) als Wärmequelle zu mind. einem der folgenden Wärmetauscher der Reaktoreinheit (102), der Konditionierungseinheit (104) oder Trenneinheit (106) führt:
- Wärmetauscher (158) in einer (Feed-)Leitung (153) zum Hauptreaktor (200) und/oder einem stromaufwärts zum Hauptreaktor (200) angeordneten Apparat (152, 154), insb. Mischer (152) oder Sättiger (154),
- Wärmetauscher (206) in der (Feed-)Leitung (116) zum Nachreaktors (210) und/oder
- Wärmetauscher (327) in der (Feed-)Leitung zur ersten Trennkolonne der Trenneinheit, und wobei mind. zwei der Wärmetauscher (158, 206, 327) zueinander parallel geschaltet sein können;
ii) vom (kühlenden) Wärmetauscher (202) des Reaktors (200) als Wärmequelle zu mind. einem der folgenden Wärmetauscher führt:
- Wärmetauscher (158) in einer (Feed-)Leitung (153) zum Hauptreaktor (200) und/oder einem stromaufwärts zum Hauptreaktor (200) angeordneten Apparat (152, 154), insb. Mischer (152) oder Sättiger (154),
- Wärmetauscher (327) in der (Feed-)Leitung (161) zur ersten Trennkolonne (320) der Trenneinheit (106), und wobei mind. zwei der Wärmetauscher (158, 206, 327) zueinander parallel geschaltet sein können; und/oder ;
iii) von einem der (kühlenden) Wärmetauscher im Kopfumlauf einer Trennkolonne der Trenneinheit (106) zu mind. einem der nachfolgenden (heizenden) Wärmetauscher im Feed oder Sumpfumlauf eines Apparates der Trenneinheit (106) führt, insb.
- vom Wärmetauscher (344, 354) der Trennkolonnen (340, 350) zum
- Wärmetauscher (327) in der (Feed-)Leitung (161) zur ersten Trennkolonne (320).
- vom Kondensator (344, 354) im Kopfkreislauf der Trennkolonne (340, 350) als Wärmequelle zum Wärmetauscher (302) im Sumpfumlauf des Trennkessels (300).

13. Anlage nach einem der Ansprüche 11 bis oder 12, **dadurch gekennzeichnet, dass** die Trenneinheit (106) in der ersten Trennstufe (106A) in der (Feed-)Leitung (211) zum Trennkessel (300) mind. eine Druckregeleinheit (220) mit einer Kondensationseinheit (304) für das Lösungsmittel umfasst, wobei eine (Rück-)Leitung (311) für das Lösungsmittel von der mind. einen Kondensationseinheit (304) der ersten Trennstufe (106A) zur Konditionierungseinheit (104) führt.

14. Anlage nach einem der vorherigen Ansprüche, **dadurch gekennzeichnet, dass** die Reaktoreinheit einen weiteren Hauptreaktor (201) als Festbettreaktor umfasst, der
- einen ersten Strömungsweg für das Reaktionsgemisch und
- einen weiteren Strömungsweg als Medienumlauf (501) für ein Wärmetauschmittel umfasst,
und wobei stromaufwärts zu den beiden Hauptreaktoren (200, 201) in der (Zu-)Leitung eine Ventileinheit vorgesehen ist, mittels welcher der Volumenstrom der Eduktmischung zw. dem ersten Hauptreaktor (200) und dem weiteren Hauptreaktor (201) aufteilbar, durchleitbar und/oder vollständig umschaltbar ist, und wobei
beide Hauptreaktoren (200, 201)
- jeweils mit einem Wärmetauscher (202, 203) oder
- einem gemeinsamen Wärmetauscher (203)
mit dem weiteren Strömungsweg (Medienumlauf) verbunden sind.

15. Anlage nach einem der vorherigen Ansprüche, **dadurch gekennzeichnet, dass** mind. ein Wärmetauschkreislauf (250, 260) als serielle integrierte stoffbasierte EK umfasst ist, der eine Mehrzahl von Leitungsabschnitten (250.1 ... 250.10; 260.1 ... 260.13) aufweist, in den mind. die folgenden Apparate und Wärmetauscher eingebunden und über mind. einen der Leitungsabschnitte (250.1 ... 250.10; 260.1 ... 260.13) verbunden sind,
i) als Wärmetauscher und als Wärmequelle für den Wärmetauschkreislauf (250, 260)
- der (Umlauf-)Wärmetauscher (202) des Hauptreaktors (200, 201) **oder**
- der (Kopf-)Wärmetauscher (304/327) in der (Kopf-)Leitung (163) des Trennkessels (300), jeweils in integrierter stoffbasierter EK mit mind. einer der nachstehenden Leitungen mit dem darin geführten Edukt- oder Stoffstrom als Wärmesenke für den Wärmetauschkreislauf (250, 260):
a) der (Feed-)Leitung (161) zur ersten Trennkolonne (320), stromabwärts zur Druckregeleinheit (222),
b) der (Feed-)Leitung (116) zum Nachreaktor (210), insb. auf der Saugseite einer dort eingebundenen Pumpe (205),
c) der (Feed-)Leitung (153) stromaufwärts zum Reaktor (200), insb. einem hierzu stromaufwärts befindlichen Mischbehälter (154), oder der (Rück-)Leitung (311) zur Konditionierungseinheit (104) stromaufwärts zum Mischer (152);
ii) als Apparate mind. der Hauptreaktor (200), insb. der Hauptreaktor (200) und der Nachreaktor (210), mind. ein Trennkessel (300), mind. ein Sammelbehälter (310) der ersten Trennstufe (106A), und wobei
iii) die folgenden Leitungen als Leitungsabschnitte des Kreislaufs (250, 260) integriert sind:
a) ein (der erste) Leitungsabschnitt (250.1, 260.1), welcher der (Kopf-)Leitung (163) des mind. einen Trennkessels (300) entspricht und/oder
b) ein (letzter) Leitungsabschnitt (250.12, 260.5), welcher der (Feed-)Leitung (162) zu dem mind. einen Sammeltank (310) entspricht.

16. Verfahren zur katalytischen Hydrierung von Methylendianilin (MDA; Edukt1) mit einem Wasserstoffgeber (Edukt2), insb. einem gasförmigen Wasserstoffgeber, vorzugsweises Wasserstoff (H2), wobei die Herstellung mittels einer industriellen Anlage (100) erfolgt, **dadurch gekennzeichnet, dass**
die Anlage (100) nach mindestens einem der vorstehenden Vorrichtungsansprüche ausgebildet ist, wobei der Hauptreaktor (200) bei einer Temperatur im Bereich von 80 °C bis 150 °C betrieben wird, insb. isotherm betrieben wird, und wobei durch EK:
i) vom Kondensator (304) der Trenneinheit (106) als Wärmequelle für mind. einen Wärmetauscher der Reaktoreinheit (102), der Konditionierungseinheit (104) und/oder der Trenneinheit (106) durch integrierte Energiekopplung (integrierte EK) oder direkte Energiekopplung (direkte EK) Energie im Bereich von 5 bis 100 % der verfügbaren Wärmemenge transferiert wird, insb. im Bereich von 20 bis 40 %;
ii) Wärmetauscher (202) des Medienumlaufs für den Hauptreaktor (200) der Reaktoreinheit (102) als Wärmequelle für mind. einen Wärmetauscher der Reaktoreinheit (102), der Konditionierungseinheit (104) und/oder der Trenneinheit (106) durch integrierte EK oder direkte EK Energie im Bereich von 5 bis 30 % der verfügbaren Wärmemenge transferiert wird, insb. im Bereich von 10 bis 20 % und/oder
iii) Wärmetauscher der Trenneinheit (106) untereinander durch integrierte EK oder direkte EK Energie im Bereich von 5 bis 100 % der verfügbaren Wärmemenge transferiert wird, insb. im Bereich von 30 bis 90 %.

17. Verfahren nach Anspruch 16, **dadurch gekennzeichnet, dass** die Temperatur des Eduktstroms am Einlass des Hauptreaktors (200, 201) 90 bis 140°C beträgt, idealerweise 100 bis 135 °C.

18. Verfahren nach einem der vorherigen Verfahrensansprüche, **dadurch gekennzeichnet, dass** dieses kontinuierlich, katalytisch zur Produktion von Methylenbis(cyclohexylamin) erfolgt, insb. zur Produktion von 4,4'-Diaminodicyclohexylmethan (PACM), bevorzugt 4,4'-Diaminodicyclohexylmethan (PACM) mit niedrigen Anteilen an trans/trans-Isomeren.

19. Verfahren nach einem der vorherigen Verfahrensansprüche, **dadurch gekennzeichnet, dass** der mind. eine Hauptreaktor (200) bei einem Druck im Bereich von 60 bar bis 120 bar betrieben wird, idealerweise im Bereich von 70 bis 110 bar.

20. Verfahren nach einem der vorherigen Verfahrensansprüche, **dadurch gekennzeichnet, dass** neben dem mind. einen Hauptreaktor (200) weiterhin mind. ein Nachreaktor (210) umfassend einen immobilen Katalysator vorgesehen ist, wobei der mind. eine Hauptreaktor (200) und der mind. ein Nachreaktor (210) bei demselben oder im Wesentlichen bei demselben Druck betrieben werden.

21. Verfahren nach einem der vorherigen Verfahrensansprüche, **dadurch gekennzeichnet, dass** vom Zeitpunkt t₀, dem Verfahrensstart nach Erneuerung oder Regeneration des Katalysators bis zum Zeitpunkt t₄, dem Ende des Verfahrens, bestimmt durch die Erneuerung oder Regeneration des Katalysators, die Betriebstemperatur des Hauptreaktors (200) erhöht und Temperatur im Stoffstrom im Einlass (Feed) des Nachreaktors (210) gleichgehalten oder gesenkt wird, wobei die Temperaturerhöhung oder -Absenkung linear und/oder schrittweise erfolgt.

22. Verfahren nach einem der vorherigen Verfahrensansprüche, **dadurch gekennzeichnet, dass** das MDA (Edukt1) eine Mischung eine Mischung der folgenden Monomere umfasst: 4,4' MDA, 2,4' MDA und 2,2' MDA, wobei der Anteil an 4,4' MDA vorteilhafterweise im Bereich von 75 bis 98 mol-% beträgt, idealerweise 85 bis 95 mol-%.
